(19) Europäisches Patentamt / European Patent Office / Office européen des brevets

(11) **EP 3 310 819 B1**

(12) **EUROPÄISCHE PATENTSCHRIFT**

(45) Veröffentlichungstag und Bekanntmachung des Hinweises auf die Patenterteilung:
**12.06.2019 Patentblatt 2019/24**

(21) Anmeldenummer: **16729265.5**

(22) Anmeldetag: **15.06.2016**

(51) Int Cl.:
***C08F 2/38*** $^{(2006.01)}$ ***C08F 2/42*** $^{(2006.01)}$

(86) Internationale Anmeldenummer:
**PCT/EP2016/063796**

(87) Internationale Veröffentlichungsnummer:
**WO 2016/202883 (22.12.2016 Gazette 2016/51)**

# (54) ZUSAMMENSETZUNG ZUR SOFORTBEENDIGUNG EINER RADIKALISCHEN POLYMERISATION

COMPOSITION FOR IMMEDIATELY ENDING RADICAL POLYMERIZATION

COMPOSITION POUR L’ARRÊT IMMÉDIAT D’UNE POLYMÉRISATION RADICALAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.06.2015 DE 102015211083**
**17.06.2015 US 201562180625 P**

(43) Veröffentlichungstag der Anmeldung:
**25.04.2018 Patentblatt 2018/17**

(73) Patentinhaber: **BASF SE**
**67056 Ludwigshafen am Rhein (DE)**

(72) Erfinder:
- **MARGUERRE, Ann-Kathrin**
  **68167 Mannheim (DE)**
- **RAITH, Christian**
  **68167 Mannheim (DE)**
- **KUJAT, Christof**
  **67434 Neustadt (DE)**
- **MARTIN, Friedrich-Georg**
  **69124 Heidelberg (DE)**
- **BADINE, Daher Michael**
  **68165 Mannheim (DE)**
- **GEYER, Karolin**
  **67061 Ludwigshafen (DE)**
- **SCHAEFER, Ansgar**
  **76199 Karlsruhe (DE)**
- **NESTLE, Nikolaus**
  **69123 Heidelberg (DE)**
- **CETINKAYA, Murat**
  **2582 XZ Den Haag (NL)**
- **SCHREINER, Eduard**
  **68169 Mannheim (DE)**
- **WEILER, Reiner**
  **67361 Freisbach (DE)**
- **ZUROWSKI, Peter**
  **76829 Landau (DE)**

(74) Vertreter: **BASF IP Association**
**BASF SE**
**ZRX-C6**
**67056 Ludwigshafen (DE)**

(56) Entgegenhaltungen:
**EP-A1- 2 017 293**
**WO-A2-99/21893**
**DE-A1-102013 000 128**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 310 819 B1

## Beschreibung

Die Erfindung betrifft eine Zusammensetzung zur Sofortbeendigung einer radikalischen Polymerisation, deren Verwendung zur Stabilisierung von radikalisch polymerisierbaren Monomeren gegen radikalische Polymerisation und ein Verfahren zur Sofortbeendigung von radikalischen Polymerisationen.

Die vorzeitige Polymerisation von Acrylmonomeren im Sinne einer Durchgehreaktion kann wegen der großen Reaktionsenthalpie zum Verdampfen von Monomeren führen. Durch den damit verbundenen Druckanstieg können z.B. Aufbewahrungsbehälter bersten und sich dabei entstehende Dämpfe entzünden, so dass es bei einem solchen Ereignis zur Explosion kommen kann. Dies führt zur Gefährdung von Personen, Sachgütern und der Umwelt. Als sicherheitstechnisch besonders kritisch ist dabei die Acrylsäure einzustufen, da sie aufgrund der großen Vinylmonomeren-Dichte eine hohe spezifische Reaktionsenthalpie aufweist und mit einem Siedepunkt von 141 °C leicht während der Durchgehreaktion verdampft. Eine Möglichkeit, die beginnende Durchgehreaktion zu beenden, ist die Zugabe von Inhibitoren zum polymerisierenden System.

Aus der deutschen Patentanmeldung DE 100 36 959 A1 ist die Verwendung von Inhibitoren, wie Hydrochinon, Hydrochinonmonomethylether, p-Benzochinon, p-Nitrosophenol (PNP), Phenothiazin (PTZ), 4-Hydroxy-2,2,6,6-tetramethyl-1-oxyl-piperidin (OH-Tempo) oder Methylenblau zur Stabilisierung von Acrylsäure und Methacrylsäure gegen unerwünschte radikalische Polymerisation bekannt.

Phenothiazine sind effektive Inhibitoren der radikalischen Polymerisation. Phenothiazine weisen allerdings eine sehr geringe Löslichkeit in gängigen Lösungsmitteln auf. Die Verwendung stark verdünnter Lösungen ist im Notfall, wenn große Mengen Inhibitor in kurzer Zeit eingebracht werden müssen, jedoch nachteilig.

Die EP 2017293 A1 offenbart Gemische, die einen Inhibitor der radikalischen Polymerisation und eine ionische Flüssigkeit enthalten, und ihre Verwendung zur Stabilisierung radikalisch polymerisierbarer Monomere. Obgleich ionische Flüssigkeiten ein hohes Lösevermögen für Phenothiazine aufweisen, ist die hohe Viskosität der Lösungen nachteilig für das schnelle Einmischen in Monomere im Notfall.

Die WO 99/21893 offenbart ein Verfahren zur Sofortbeendigung radikalischer Polymerisationen durch Zusatz einer Phenothiazin enthaltenden Lösung, deren Lösungsmittel zu wenigstens 50% seines Gewichts aus einem N-Alkylpyrrolidon besteht. Es ist wünschenswert, über alternative Lösungsmittel zu verfügen, da N-Alkylpyrrolidone, wie z.B. N-Methylpyrrolidon (NMP), als toxikologisch bedenklich einzustufen sind.

Der Erfindung liegt die Aufgabe zugrunde, eine Zusammensetzung zum schnellen Stoppen einer radikalischen Polymerisation bereitzustellen, die eine hohe Inhibitorkonzentration und angemessene Viskosität aufweist und deren Lösungsmittelsystem inert gegenüber Monomeren wie Acrylsäure ist.

Die Aufgabe wird gelöst durch eine Zusammensetzung, die a) einen unter Phenothiazinen ausgewählten Inhibitor der radikalischen Polymerisation, b) ein aprotisches Lösungsmittel und c) eine ionische Flüssigkeit umfasst.

Die Erfindung betrifft ferner ein Verfahren zur Sofortbeendigung von radikalischen Polymerisationen, wobei man die oben genannte Zusammensetzung einem radikalisch polymerisierenden System zusetzt.

Die erfindungsgemäße Zusammensetzung enthält vorzugsweise über einen Temperaturbereich von 0 bis 40 °C hinweg keinen ungelösten Inhibitor. Sie liegt (bei Normaldruck) über einen Temperaturbereich von 0 bis 40 °C hinweg vorzugsweise einphasig vor, d.h. als homogenes Gemisch ohne Phasenseparation. Sie kann jedoch auch in Form von zweiphasig flüssigen Systemen vorliegen.

Die erfindungsgemäße Zusammensetzung enthält mindestens einen, insbesondere genau einen, Inhibitor der radikalischen Polymerisation, der unter Phenothiazinen ausgewählt ist.

Bekanntermaßen handelt es sich bei der radikalischen Polymerisation um eine Kettenreaktion, bei der im Initiierungsschritt Radikale gebildet werden, an die sich die zu polymerisierenden Monomere so lange anlagern, bis ein Abbruch durch Kombination oder Disproportionierung zweier Makroradikale oder deren Reaktion mit Reglersubstanzen oder Verunreinigungen wie Sauerstoff eintritt (vgl. a. Römpp Online, 2007, »Radikalische Polymerisation«).

Bekanntermaßen wirken Inhibitoren als Radikalfänger und hemmen so die Bildung von Radikalen im Initiierungsschritt der Kettenreaktion (vgl. a. Römpp Online, 2007, »Inhibierung«).

Phenothiazine sind effektive Inhibitoren der radikalischen Polymerisation. Geeignete Phenothiazine sind solche der allgemeinen Formel I:

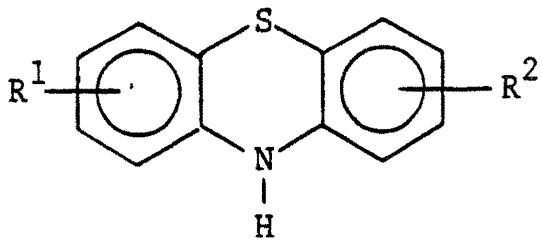

(I)

worin $R^1$ und $R^2$ unabhängig voneinander für Wasserstoff, $C_6$-$C_{10}$-Aryl, $C_7$-$C_{11}$-Aralkyl, $C_7$-$C_{16}$-Alkaryl oder $C_1$-$C_{12}$-Alkyl stehen.

Geeignete Phenothiazine sind Phenothiazin, 2-Methylphenothiazin, 2-Octylphenothiazin, 2-Nonylphenothiazin, 2,8-Dimethylphenothiazin, 3,7-Dimethylphenothiazin, 3,7-Diethylphenothiazin, 3,7-Dibutylphenothiazin, 3,7-Dioctylphenothiazin und 2,8-Dioctylphenothiazin, 3,7-Dinonylphenothiazin, 2,8-Dinonylphenothiazin, 2-(α,α-Dimethylbenzyl)phenothiazin, 3,7-Bis(α,α-dimethylbenzyl)phenothiazin und 2,8-Bis(α,α-dimethylbenzyl)phenothiazin.

Als Inhibitor wird in der erfindungsgemäßen Zusammensetzung vorzugsweise Phenothiazin ($R^1$ = $R^2$ = H) verwendet.

Die Zusammensetzung enthält ein aprotisches Lösungsmittel oder eine Kombination aprotischer Lösungsmittel. Das aprotische Lösungsmittel enthält keine aciden Wasserstoffatome, d.h. es verfügt nicht über an ein Sauerstoffatom oder Stickstoffatom gebundene Wasserstoffatome.

Das aprotische Lösungsmittel weist im Allgemeinen einen Siedepunkt von mindestens 80 °C bei Normaldruck auf, vorzugsweise von mindestens 100 °C und insbesondere von mindestens 150 °C.

Vorzugsweise umfasst das aprotische Lösungsmittel keine von Kohlenstoff, Sauerstoff, Stickstoff und/oder Wasserstoff verschiedenen Atome; insbesondere keine von Kohlenstoff, Sauerstoff und/oder Wasserstoff verschiedenen Atome.

Üblicherweise weist das aprotische Lösungsmittel eine relative statische Permittivität $\varepsilon_r$ (auch als Dielektrizitätskonstante, Dielektrizitätszahl oder Permittivitätszahl bezeichnet) als flüssige Reinsubstanz im Bereich von 3 bis 50, vorzugsweise 4 bis 38, besonders bevorzugt 5 bis 20, bei einer Temperatur von 293,15 K und einem Druck von $1{,}0133 \cdot 10^5$ Pa (= Normaldruck) (die relative statische Permittivität von Vakuum = 1) auf. Eine geeignete Quelle mit Angaben zu relativen statischen Permittivitäten von geeigneten relevanten aprotischen Substanzen ist z. B. das HANDBOOK of CHEMISTRY and PHYSICS, 92th Edition (2010-2011), CRC PRESS.

Alternativ oder zusätzlich weist das aprotische Lösungsmittel eine Lage im Hansen-Löslichkeitsraum auf, die gekennzeichnet ist durch

$$\sqrt{4(\delta_D - 17)^2 + (\delta_P - 11)^2 + (\delta_H - 6)^2} \leq 9 \text{ (Formel 1).}$$

Der Wert wird im Folgenden auch als Löslichkeitsdistanz $R_a$ bezeichnet. Die Definition und die Berechnung der Löslichkeitsparameter im dreidimensionalen Hansen-Löslichkeitsraum sind beschrieben in: C. M. Hansen: "The three dimensional solubility parameters" J. Paint Technol. 39, 105 (1967). Gemäß diesem Hansenraum kennzeichnet $\delta_D$ die LONDON-Dispersionskräfte, kennzeichnet $\delta_P$ die Debye-Interaktionskräfte zwischen permanenten Dipolen sowie die Keesom-Interaktionskräfte zwischen induzierten und permanenten Dipolen und kennzeichnet $\delta_H$ die spezifischen Interaktionskräfte (Wasserstoffbindungen, Säure/Base, Donator/Akzeptor usw.). Die Einheit der Anteile der Parameter ist jeweils [$MPa^{1/2}$].

Die Hansen-Löslichkeitsparameter für viele Lösungsmittel sind in Standardwerken tabelliert, wie Hansen Solubility Parameters: A User's Handbook, C. M. Hansen, 2007, 2nd Edition. Es kann auch bekannte Modellier-Software, wie beispielsweise HSPIP 3.1.14 (3rd Edition), entwickelt und vertrieben von C. M. Hansen, verwendet werden, um die Hansen-Löslichkeitsparameter auf Basis der chemischen Struktur des Lösungsmittels zu berechnen. Die hier verwendeten Hansen-Löslichkeitsparameter beziehen sich auf Raumtemperatur, etwa 23 °C.

Beispielhaft sind in der folgenden Tabelle die jeweiligen Anteile der Löslichkeitsparameter verschiedener Lösungsmittel ($\delta_D$, $\delta_P$, $\delta_H$) und die aus Formel 1 berechneten Löslichkeitsdistanzen aufgeführt.

Tabelle 1: Hansen-Löslichkeitsparameter und relative statische Permittivitäten $\varepsilon_r$ für verschiedene Lösungsmittel

| Lösungsmittel | $\delta_D$ | $\delta_P$ | $\delta_H$ | $R_a$ | $\varepsilon_r$ bei 20 °C |
|---|---|---|---|---|---|
| DMSO | 18,4 | 16,4 | 10,2 | 7,4 | 47,3 |

(fortgesetzt)

| Lösungsmittel | $\delta_D$ | $\delta_P$ | $\delta_H$ | $R_a$ | $\varepsilon_r$ bei 20 °C |
|---|---|---|---|---|---|
| Sulfolan | 17,8 | 17,4 | 8,7 | 7,1 | 42,7 |
| Benzonitril | 18,8 | 12,0 | 3,3 | 4,6 | 25,7 |
| Dimethylsuccinat | 16,1 | 7,7 | 8,8 | 4,7 | 7,3 |
| Methylbenzoat | 18,9 | 8,2 | 4,7 | 4,9 | 6,7 |
| γ-Butyrolacton | 18,0 | 16,6 | 7,4 | 6,1 | 41,4 |
| Acetonitril | 15,3 | 18,0 | 6,1 | 7,8 | 36,8 |
| Cyclohexanon | 17,8 | 8,4 | 5,1 | 3,2 | 16,1 |
| Methylbutylketon | 15,3 | 6,1 | 4,1 | 6,3 | 14,5 |
| Morpholin | 18,0 | 4,9 | 11,0 | 8,1 | 7,8 |
| Dibutyladipat (Cetiol B) | 16,4 | 4,3 | 5,9 | 6,8 | 3,0 |
| Di-(2-ethylhexyl)adipat (Plastomoll DOA) | 16,2 | 4,6 | 7,7 | 6,8 | 2,1 |
| $C_{10}$-Fettsäure-dimethylamid (Agnique AMD 10) | 16,6 | 6,7 | 5,7 | 4,4 | 13,8 |
| N-Methylimidazol | 19,7 | 15,6 | 11,2 | 8,8 | 32,0 |
| Dipropylenglycol-dimethylether (Proglyme) | 15,5 | 4,6 | 6,1 | 7,1 | 10,4 |
| N-Methylpyrrolidon | 16,8 | 2,8 | 6,7 | 8,2 | 32,8 |

Als aprotische Lösungsmittel eignen sich aromatische Kohlenwasserstoffe, aliphatische Kohlenwasserstoffe, Ether, Ester (einschließlich cyclischer Ester), Amide (einschließlich cyclischer Amide), Nitrile, Acetale oder Mischungen davon.

Beispiele für aromatische Kohlenwasserstoffe sind Benzol, Biphenyl, o-Terphenyl, m-Terphenyl, Naphthalin, ein- oder mehrfach $C_1$-$C_{20}$-Alkyl-substituierte aromatische Kohlenwasserstoffe wie Toluol, Xylol, Dodecylbenzol, Tetradecylbenzol, Hexadecylbenzol, Methylnaphthalin, Diisopropylnaphthalin, Hexylnaphthalin oder Decylnaphthalin. Geeignet sind auch Gemische der vorgenannten aromatischen Kohlenwasserstoffe, insbesondere auch technische Aromatengemische.

Beispiele für aliphatische Kohlenwasserstoffe sind gesättigte oder ungesättigte $C_5$-$C_{40}$-Kohlenwasserstoffe, insbesondere $C_{10}$-$C_{40}$-Kohlenwasserstoffe, die verzweigt, cyclisch oder linear sind, wie n-Tetradecan, n-Pentadecan, n-Hexadecan, n-Heptadecan, n-Octadecan, n-Nonadecan, n-Eicosan, n-Heneicosan, n-Docosan, n-Tricosan, n-Tetracosan, n-Pentacosan, n-Hexacosan, n-Heptacosan, n-Octacosan, Mineralöle oder hochdruckhydrierte Mineralöle (so genannte Weißöle). Geeignet sind auch Gemische der vorgenannten aliphatischen Kohlenwasserstoffe.

Beispiele für Ester sind $C_1$-$C_{40}$-Alkylester von $C_2$-$C_{40}$-Alkansäuren, Ester von $C_6$-$C_{24}$-Alkoholen mit aromatischen Carbonsäuren, Ester von $C_2$-$C_{12}$-Dicarbonsäuren mit linearen oder verzweigten Alkoholen mit 1 bis 22 Kohlenstoffatomen oder Ester von $C_2$-$C_{40}$-Alkansäuren mit Polyolen mit 2 bis 10 Kohlenstoffatomen und 2 bis 6 Hydroxylgruppen.

Hierzu zählen $C_1$-$C_{40}$-Alkylester von $C_8$-$C_{40}$-Alkansäuren oder $C_6$-$C_{40}$-Alkylester von $C_2$-$C_{40}$-Alkansäuren. Weitere Beispiele für Ester sind Ester von linearen $C_6$-$C_{24}$-Fettsäuren mit linearen $C_3$-$C_{24}$-Alkoholen, Ester von verzweigten $C_6$-$C_{13}$-Carbonsäuren mit linearen $C_6$-$C_{24}$-Fettalkoholen, Ester von linearen $C_6$-$C_{24}$-Fettsäuren mit verzweigten Alkoholen, insbesondere 2-Ethylhexanol.

Von Bedeutung sind Monoester der Fettsäuren mit Alkoholen mit 3 bis 24 C-Atomen. Bei dieser Stoffgruppe handelt es sich um die Produkte der Veresterung von Fettsäuren mit 8 bis 24 Kohlenstoffatomen wie beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckabspaltung von natürlichen Fetten und Ölen, bei der Reduktion von Aldehyden aus der Roelen'schen Oxosynthese oder der Dimerisierung von ungesättigten Fettsäuren anfallen, mit Alkoholen wie beispielsweise Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linoylalkohal, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Beispiele für Alkandisäure-dialkylester sind Di-$C_2$-$C_{32}$-alkylester von $C_4$-$C_{32}$-alkandisäuren, bevorzugt Di-$C_2$-$C_{18}$-alkylester von $C_6$-$C_{16}$-alkandisäuren. Besonders geeignet unter den Alkandisäure-dialkylestern sind Bernsteinsäuredibutylester, Adipinsäuredibutylester und Phthalsäuredibutylester, darunter insbesondere Adipinsäuredibutylester.

Weiter eignen sich Ester von linearen und/oder verzweigten Fettsäuren mit mehrwertigen Alkoholen (wie z.B. Propylenglycol, Dimerdiol oder Trimertriol), oder Triglyceride auf Basis von $C_6$-$C_{18}$-Fettsäuren, wie Pflanzenöle.

Unter Ester fallen auch cyclische Ester wie zum Beispiel gamma-Butyrolacton und delta-Valerolacton.

Beispiele für Amide sind N,N-Di-$C_1$-$C_{12}$-alkyl-$C_8$-$C_{22}$-alkylamide, wie N,N-Dimethyldecanamid, oder N,N-Dimethyldodecanamid.

Cyclische Amide sind beispielsweise N-Methylpyrrolidon, Caprolactam, Dimethylethylenharnstoff und/oder Dimethylpropylenharnstoff.

Beispiele für Ether sind Dialkylether, Alkylarylether, Diarylether und Polyolpolyether. Dialkylether sind lineare oder verzweigte, symmetrische oder unsymmetrische Dialkylether mit insgesamt zwischen 12 bis 36 Kohlenstoffatomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether, n-Hexyl-n-undecylether, Di-tert-butylether, Diiso-pentylether, Di-3-ethyldecylether, tert-Butyl-n-octylether, iso-Pentyl-n-octylether und 2-Methyl-pentyl-n-octylether.

Bevorzugt sind ferner Polyolpolyether wie Diethylenglycol-dimethylether (Diglyme), Dipropylenglycol-dimethylether (Proglyme).

Weiter eignen sich Alkylether von Glycolacetaten wie 2-Methoxyethyl-acetat, 2-Ethoxyethyl-acetat, 2-Butoxyethyl-acetat und 1-Methoxy-2-propyl-acetat.

Ein geeignetes Acetal ist Anisacetal (p-(Dimethoxymethyl)anisol).

Besonders bevorzugte aprotische Lösungsmittel sind Alkandisäure-dialkylester und Polyolpolyether, insbesondere Dipropylenglycol-dimethylether.

Die erfindungsgemäße Zusammensetzung enthält mindestens eine, insbesondere genau eine, ionische Flüssigkeit.

Vorzugsweise beträgt der Schmelzpunkt der ionischen Flüssigkeit bei Normaldruckdruck weniger als 100°C, bevorzugt weniger als 50°C und insbesondere weniger als 20°C.

Bekanntermaßen bestehen ionischen Flüssigkeiten aus organischen Kationen und organischen oder anorganischen Anionen.

Als organische Kationen kommen alle Kationen in Betracht, wie sie üblicherweise in ionischen Flüssigkeiten verwendet werden. Vorzugsweise werden die organischen Kationen unter quartären Ammonium-, Oxonium-, Sulfonium- und Phosphonium-Kationen, sowie unter Uronium-, Thiouronium- und Guanidinium-Kationen, bei denen die einfach positive Ladung über mehrere Heteroatome delokalisiert ist, ausgewählt.

Besonders bevorzugt werden quartäre Ammonium-Kationen und ganz besonders bevorzugt heterocyclische quartäre Ammonium-Kationen verwendet.

Insbesondere werden die heterocyclischen quartären Ammonium-Kationen unter Pyrrolium-, Imidazolium-, 1H-Pyrazolium-, 3H-Pyrazolium-, 4H-Pyrazolium-, 1-Pyrazolinium-, 2-Pyrazolinium-, 3-Pyrazolinium-, 2,3-Dihydro-imidazolinium-, 4,5-Dihydro-imidazolinium-, 2,5-Dihydro-imidazolinium-, Pyrrolidinium-, 1,2,4-Triazolium- (quartäres Stickstoffatom in 1-Stellung), 1,2,4-Triazolium- (quartäres Stickstoffatom in 4-Stellung), 1,2,3-Triazolium- (quartäres Stickstoffatom in 1-Stellung), 1,2,3-Triazolium- (quartäres Stickstoffatom in 4-Stellung), Oxazolium-, Isoxazolium-, Thiazolium-, Isothiazolium-, Pyridinium-, Pyridazinium-, Pyrimidinium-, Piperidinium-, Morpholinium-, Pyrazinium-, Indolium-, Chinolinium-, Isochinolinium-, Chinoxalinium-und Indolinium-Kationen ausgewählt.

Die vorstehend beschriebenen organischen Kationen sind an sich bekannte Spezies, die beispielsweise in den deutschen Patentanmeldungen DE 10 2005 055 815 A, Seite 6, Absatz [0033], bis Seite 15, Absatz [0074], DE 10 2005 035 103 A1 , Seite 3, Absatz [0014], bis Seite 10, Absatz [0051], und DE 103 25 050 A1, der die Seiten 2 und 3 übergreifende Absatz [0006] in Verbindung mit Seite 3, Absatz [0011], bis Seite 5, Absatz [0020], im Detail beschrieben werden. Auf die aufgeführten Passagen der deutschen Patentanmeldungen wird zu Zwecken der näheren Erläuterung der vorliegenden Erfindung ausdrücklich Bezug genommen.

Geeignete organische Kationen umfassen vorzugsweise Ammoniumionen der Formel (II)

$$N^+R^1R^2R^3R^4 \qquad (II),$$

wobei $R^1$, $R^2$, $R^3$ und $R^4$ unabhängig voneinander Alkylreste sind, die mit heterofunktionellen Gruppen substituiert sein können, und die untereinander aliphatische Ringsysteme bilden können.

Vorzugsweise sind $R^1$, $R^2$, $R^3$ und $R^4$ unabhängig voneinander Alkyl und/oder Hydroxyalkyl. Insbesondere bevorzugt sind $R^1$, $R^2$, $R^3$ und $R^4$ unabhängig voneinander $C_1$-$C_{20}$ Alkyl und/oder $C_1$-$C_{20}$ Hydroxyalkyl. Ganz besonders bevorzugt sind $R^1$, $R^2$, $R^3$ und $R^4$ unabhängig voneinander $C_1$-$C_8$ Alkyl und/oder $C_1$-$C_8$ Hydroxyalkyl. Beispiele sind Tetrabutylammonium (TBA) oder Cholinium (N,N,N-Trimethyl-N-hydroxyethylammonium).

In einer weiteren bevorzugten Ausführungsform sind $R^1$, $R^2$, $R^3$ und $R^4$ unabhängig voneinander Alkylreste, die mit heterofunktionellen Gruppen substituiert sein können, und die untereinander aliphatische Ringsysteme bilden.

Beispiele sind N,N-Di-$C_1$-$C_{12}$-pyrrolidinium, 5-Aza-spiro[4.4]nonan oder N,N-Dimethylpyrrolidinium.

Geeignete organische Kationen umfassen weiterhin ein Imidazoliumion der Formel (III)

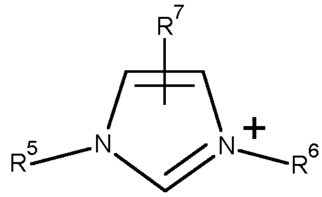

(III),

wobei $R^5$ für Wasserstoff oder Alkyl steht, $R^6$ für Alkyl und $R^7$ für Wasserstoff oder Alkyl steht. Der Alkylrest kann linear, cyclisch oder verzweigt sein. Bevorzugt ist $R^5$ Wasserstoff oder $C_1$-$C_{20}$-Alkyl, $R^6$ $C_1$-$C_{20}$-Alkyl, und $R^7$ H oder $C_1$-$C_6$-Alkyl. Besonders bevorzugt ist $R^5$ Wasserstoff oder $C_1$-$C_6$-Alkyl, $R^6$ $C_1$-$C_6$-Alkyl, und $R^7$ H oder $C_1$-$C_6$ Alkyl. $R^7$ ist bevorzugt H oder Methyl, insbesondere H. Beispiele sind N-Ethyl-N'-methylimidazolium (EMIM), N-Methylimidazolium (MEHIM), N-Butyl-N'-methylimidazolium (BMIM), N-Ethyl-N'-ethyl-imidazolium (EEIM), N-n-Propyl-N1-n-propyl-imidazolium (PPIM).

Geeignete organische Kationen umfassen weiterhin N-substituierte Pyridiniumderivate, wie N-Alkylpyridinium, wobei der Alkylrest bevorzugt ein $C_1$-$C_{12}$-Alkylrest, insbesondere ein $C_1$-$C_6$-Alkylrest, ist.

Geeignete organische Kationen umfassen weiterhin N,N'-disubstitierte Pyrazoliumderivate, wie ein N,N'-Dialkylpyrazoliumderivat, wobei der Alkylrest bevorzugt ein $C_1$-$C_{12}$-Alkylrest, insbesondere ein $C_1$-$C_6$-Alkylrest, ist. Das Dialkylpyrazoliumderivat kann optional mit einem $C_1$-$C_4$-Alkyl substituiert sein, wie zum Beispiel 1,2,5-Trimethylpyrazolium.

Geeignete organische Kationen umfassen weiterhin ein Guanidiniumderivat, wie Guanidinium, Hexamethylguanidinium, Arginin-Kation, oder Kreatinium.

Von den vorstehend beschriebenen organischen Kationen werden vor allem Imidazolium-Kationen, insbesondere das 1-Ethyl-3-methylimidazolium-Kation (EMIM) oder das 1-Butyl-3-methylimidazolium-Kation (BMIM), worin sich der quartäre Stickstoff jeweils in 1-Stellung befindet, verwendet.

Als anorganische und organische Anionen kommen alle Anionen in Betracht, wie sie üblicherweise in ionischen Flüssigkeiten verwendet werden. Beispiele geeigneter Anionen werden in den deutschen Patentanmeldungen DE 10 2005 055 815 A, Seite 2, Absatz [006] in Verbindung mit Seite 15, Absatz [0075], bis Seite 17, Absatz [0088], und DE 103 25 050 A1 , der die Seiten 2 und 3 übergreifende Absatz [0006] in Verbindung mit Seite 5, Absatz [0021], im Detail beschrieben. Auf die aufgeführten Passagen der deutschen Patentanmeldungen wird zu Zwecken der näheren Erläuterung der vorliegenden Erfindung ausdrücklich Bezug genommen.

Zum Beispiel umfasst das Anion ein Carboxylat, Sulfonat, Sulfat, Phosphonat, Phosphat, Halogen, Bis(trifluorosulfonyl)imid, Aluminumtetrachlorid, Phosphorfluorid (wie Phosphorhexafluorid), oder Dicyanimid, oder ein Gemisch davon.

Bevorzugte Anionen sind Carboxylate, Sulfate, Alkylsulfonate, Halogenide (wie lodid oder Chlorid), Phosphonate, Phosphate, Bis(trifluorsulfonyl)imid, oder Dicyanimid (2-Cyanoguanidin). Besonders bevorzugt sind Carboxylate, Sulfate und Alkylsulfonate, darunter insbesondere Alkylcarboxylate, Polyether-haltige Carboxylate, Alkylsulfate und Alkylsulfonate.

Geeignete Carboxylate sind $C_1$-$C_{30}$-Alkylcarboxylate, Polyether-haltige Carboxylate, Arylcarboxylate und Polycarboxylate.

Alkylcarboxylate sind z.B. Acetat, Propionat, Hexanoat, 2-Ethylhexanoat, Heptanoat, Octanoat, Isononanoat, Decanoat, Laurat, Oleat, Palmitat, Stearat, oder Octadecanoat.

Bevorzugte Polyether-haltige Carboxylate entsprechen der Formel:

$$R^aO(CH_2CH_2O)_nCH_2CO_2^-$$

worin n für eine ganze Zahl von 0 bis 3 und $R^a$ für $C_1$-$C_{14}$-Alkyl oder $CH_2CO_2^-$ steht. Ein geeignetes Beispiel ist [2-(2-Methoxyethoxy)ethoxy]acetat.

Bevorzugte Polycarboxylate sind aliphatische Di- und Tricarboxylate mit 2 bis 32 Kohlenstoffatomen, wie die Anionen von Aconitsäure, Adipinsäure, Citronensäure, Fumarsäure, Glutarsäure, Oxoglutarsäure, Maleinsäure, Äpfelsäure, Malonsäure, Oxalsäure, Sebacinsäure, Bernsteinsäure, Weinsäure.

Bevorzugte Arylcarboxylate sind die Anionen von Benzoesäure, Zimtsäure oder Hippursäure.

Geeignete Alkylsulfonate sind $C_1$-$C_{20}$-Alkylsulfonate, insbesondere $C_1$-$C_{10}$-Alkylsulfonate, wie Ethansulfonat oder Octansulfonat.

Geeignete Sulfate sind solche der Formel $R^c-OSO_3^-$, worin $R^c$ für $C_1$-$C_{18}$-Alkyl oder $C_6$-$C_{12}$-Aryl, vorzugsweise $C_1$-$C_8$-Alkyl steht. Ein geeignetes Beispiel ist Ethylsulfat.

Geeignete Phosphate sind $C_1$-$C_{10}$-Dialkylphosphate, wie Dimethylphosphat oder Dibutylphosphat.

Geeignete Halogenide sind Chlorid, Bromid oder Iodid, vorzugsweise Chlorid.

Darüber hinaus kommen die Anionen von radikalisch polymerisierbaren, olefinisch ungesättigten Säuren, vorzugsweise die Anionen von radikalisch polymerisierbaren, Vinylgruppen enthaltenden Säuren, in Betracht.

Beispiele besonders geeigneter Anionen sind die Anionen von Acrylsäure, Methacrylsäure, Ethacrylsäure, Chloracrylsäure, Cyanacrylsäure, Vinylessigsäure, Vinylphosphonsäure, Vinylsulfonsäure und Vinylbenzol-2-, -3- und 4-sulfonsäure, insbesondere von Acrylsäure und Methacrylsäure.

Die ionischen Flüssigkeiten können nach bekannten Verfahren hergestellt werden, z.B. wie beschrieben in Wasserscheid and Welton, Ionic liquids in synthesis, 2nd Edition, 2007, Wiley-VCH, oder der WO 2008/135482.

Die ionischen Flüssigkeiten können aus beliebigen Kombinationen der vorstehend beschriebenen organischen Kationen und organischen oder anorganischen Anionen zusammengesetzt sein, solange die Kombination eines bestimmten Kations mit einem bestimmten Anion nicht zu unerwünschten chemischen Reaktionen oder physikalischen Phasenumwandlungen wie den Bildung von Niederschlägen oder Phasentrennung führt, was aber der Fachmann anhand seines allgemeinen Fachwissens gegebenenfalls unter Zuhilfenahme einiger weniger orientierender Versuche leicht vorhersagen und daher vermeiden kann.

Besonders geeignete ionische Flüssigkeiten sind Tetrabutylammoniumstearat, Tetrabutylammonium [2-(2-methoxyethoxy)ethoxy]acetat, N-Ethyl-N'-methylimidazolium chlorid (EMIM Cl, z.B. Basionics ST 80), N-Butyl-N'-methylimidazolium chloride (BMIM Cl, z.B. Basionics ST 70), N-Ethyl-N'-methylimidazolium thiocyanate (EMIM SCN, z.B. Basionics VS 01), N-Ethyl-N'-methylimidazolium tetrafluoroborate (EMIM $BF_4$, z.B. Basionics EE 03), N-Ethyl-N'-methylimidazoliumacetat (EMIM OAc, z.B. Basionics BC 01), N-Ethyl-N'-methylimidazoliumisononanoat, N-Ethyl-N'-methylimidazoliumoctanoat, N-Ethyl-N'-methylimidazoliummethansulfonat (EMIM $MeSO_3$, z.B. Basionics ST 35), N-Ethyl-N'-methylimidazoliumdicyanamid (EMIM DCA, z.B. Basionics VS 03), N-Ethyl-N'-methylimidazoliumdiethylphosphat (EMIM DEP, z.B. Basionics LQ 11), N-Ethyl-N'-methylimidazoliumtriflourmethansulfonat (EMIM Otf, z.B. Basionics VS 11), N-Ethyl-N'-methylimidazoliumbis(trifluormethansulfonyl)imid (EMIM TFSI, z.B. Basionics HP 01), Tris(2-hydroxyethyl)methylammoniummethylsulfat (MTEOA MeOSO3, z.B.Basionics FS 01), N-Ethyl-N'-methylimidazoliumdibutylphosphat, N-Ethyl-N'-methylimidazolium ethylsulfat (EMIM $EtOSO_3$, z.B. Basionics LQ 01), N-Butyl-N'-methylimidazoliumacetat (BMIM Acetat, z.B. Basionics BC02), N-Ethyl-N'-ethylimidazoliumpropionat, N-Propyl-N'-propylimidazoliumacetat, Choliniumoctanoat und Choliniumformiat.

Vorzugsweise enthält die erfindungsgemäße Zusammensetzung wenigstens 20 Gew.%, insbesondere wenigstens 30 Gew.-%, meist 30 bis 60 Gew.-%, zum Beispiel 40 bis 55 Gew.-%, Inhibitor der radikalischen Polymerisation, bezogen auf das Gesamtgewicht der Komponenten a), b) und c).

Das Gewichtsverhältnis von aprotischem Lösungsmittel und ionischer Flüssigkeit in der erfindungsgemäßen Zusammensetzung liegt vorzugsweise in einem Bereich von 100 : 1 bis 1 : 10, insbesondere 90 : 10 bis 60 : 40. Ganz besonders bevorzugt ist ein Verhältnis von aprotischem Lösungsmittel zu ionischer Flüssigkeit von 85 : 15 bis 75 : 25.

Die Löslichkeit von Phenothiazin in der Kombination des aprotischen Lösungsmitttels und der ionischen Flüssigkeit ist mindestens etwa so hoch wie seine gewichtsgemittelte Löslichkeit. Die gewichtsgemittelte Löslichkeit kann berechnet werden, indem man die Löslichkeit von Phenothiazin im aprotischen Lösungsmittel mit dem Gewichtsanteil des aprotischen Lösungsmittels in der Lösungsmittelkombination multipliziert und die Löslichkeit des Phenothiazins in der ionischen Flüssigkeit mit dem Gewichtsanteil der ionischen Flüssigkeit in der Lösungsmittelkombination multipliziert und die Produkte addiert. In einigen Fällen kommt es vorteilhafterweise zu einem löslichkeitssteigerndem Effekt, wobei die tatsächliche Löslichkeit höher ist als die gewichtsgemittelte Löslichkeit.

Weiterhin weist die Kombination der Komponenten b), c) und Phenothiazin eine geringere Viskosität auf als Lösungen von Phenothiazin nur in Komponente c). Dies ist insbesondere beim Einmischen der Zusammensetzung in Monomere bei Einsetzen einer radikalischen Polymerisation von Vorteil.

Die Herstellung der erfindungsgemäßen Zusammensetzung kann beispielsweise so erfolgen, dass die vorstehend beschriebenen Komponenten a), b) und c) miteinander vermischt werden, wonach das resultierende Gemisch homogenisiert wird. Methodisch gesehen kann dieses Herstellungsverfahren mit Hilfe üblicher und bekannter Vorrichtungen zum Vermischen von Flüssigkeiten oder Flüssigkeiten und Feststoffen, wie Rührkessel, Extruder, Ultraturrax, Inline-Dissolver, Homogenisierungsdüsen oder Gegenstrommischer, durchgeführt werden.

Die Zusammensetzung kann weitere Inhibitoren enthalten, z.B. phenolische Inhibitoren, wie, 4-Methoxyphenol, 4-tert-Butyl-Brenzcatechin, Hydrochinon oder 2,6-Di-tert-butyl-4-methylphenol (Kerobit BHT),
N-Oxylverbindungen, wie 2,2,6,6,-Tetramethylpiperazin-1-oxyl (TEMPO), 4-Hydroxy-2,2,6,6,-Tetramethylpiperazin-1-oxyl (HO-TEMPO) und 4-Oxo-2,2,6,6,-tetramethylpiperazin-1-oxyl (Oxo-TEMPO),
aromatische Amine wie Diphenylamin, para-Phenylendiamin oder para-Phenylendiamin-Derivate, wie z.B. N,N'-di-sec-butyl-para-phenylendiamin (Kerobit BPD), oder
organische Nitrosoverbindungen oder Mischungen davon.

Als Nitroxyl-Radikale (auch als N-Oxyl-Radikale bezeichnet) kommen insbesondere diejenigen in Betracht, die sich von einem sekundären Amin ableiten, welches keine Wasserstoffatome an den α-C-Atomen trägt (d.h., die N-Oxyl-Gruppen leiten sich von entsprechenden sekundären Aminogruppen ab). Unter diesen eignen sich vor allem jene N-Oxyl-Radikale, die in der EP-A 135280, der älteren Anmeldung DE-A 19651307, der US-A 5,322,912, der US-A 5,412,047, der US-A 4,581,429, der DE-A 1618141, der CN-A 1052847, der US-A 4,670,131, der US-A 5,322,960, der älteren Anmeldung DE-A 19602539, der EP-A 765856 und der JP-A 5/320217 genannt sind.

Solche geeigneten, sich von einem sekundären Amin ableitenden, stabilen N-Oxyl-Radikale sind z.B. jene der Formel (IV):

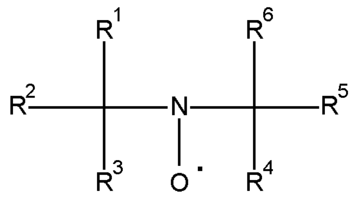

(IV)

mit $R^1$, $R^2$, $R^5$ und $R^6$ =

dieselben oder verschiedene gerad- oder verzweigtkettige, gegebenenfalls substituierte Alkylgruppen und

$R^3$ und $R^4$ =

dieselben oder verschiedene gerad- oder verzweigtkettige, gegebenenfalls substituierte Alkylgruppen oder

$R^3CNCR^4$ =

eine, gegebenenfalls substituierte, zyklische Struktur.

Als geeignete Verbindungen IV kommen insbesondere jene in Betracht, die in der EP-A 135 280, der älteren Anmeldung DE-A 19651307, der US-A 5,322,912, der US-A 5,412,047, der US-A 4,581,429, der DE-A 16 18 141, CN-A 1052847, US-A 4,670,131, US-A 5,322,960 sowie der älteren Anmeldung DE-A 19602539 genannt sind.

Beispiele dafür sind jene stabilen N-Oxyl-Radikale der allgemeinen Formel (IV), bei welchen $R^1$, $R^2$, $R^5$ und $R^6$ für (gleiche oder verschiedene) $C_1$- bis $C_4$-Alkylgruppen wie Methyl-, Ethyl-, n-Propyl-, iso-Propyl-, n-Butyl-, iso-Butyl oder tert.-Butyl-, lineares oder verzweigtes Pentyl-, Phenyl- oder substituierte Gruppen hiervon und $R^3$ und $R^4$ für (gleiche oder verschiedene) $C_1$- bis $C_4$-Alkylgruppen wie Methyl-, Ethyl-, n-Propyl-, iso-Propyl-, n-Butyl-, iso-Butyl- oder tert.-Butyl-, lineares oder verzweigtes Pentyl-, substituierte Gruppen hiervon oder gemeinsam mit CNC die zyklische Struktur

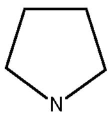 oder 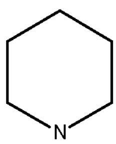 oder 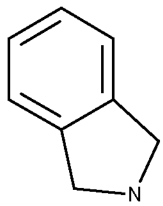 oder 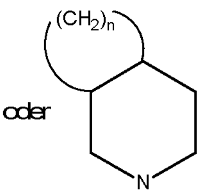 oder 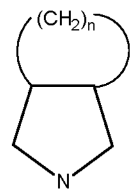

mit n gleich einer ganzen Zahl von 1 bis 10 (häufig 1 bis 6), einschließlich substituierter derartiger zyklischer Strukturen, stehen. Als beispielhafte Vertreter seien 2,2,6,6-Tetramethyl-1-oxyl-piperidin, 2,2,5,5-Tetramethyl-I-oxyl-pyrrolidin und 4-Oxo-2,2,6,6-tetramethyl-1-oxyl-piperidin genannt.

Die N-Oxyl-Radikale (IV) lassen sich aus den entsprechenden sekundären Aminen durch Oxidation, z.B. mit Wasserstoffperoxid, herstellen. In der Regel sind sie als Reinsubstanz darstellbar.

Zu den geeigneten N-Oxyl-Radikalen (IV) zählen insbesondere Piperidin- oder Pyrrolidin-N-Oxyle und Di-N-Oxyle der nachstehenden allgemeinen Formeln (V) bis (XII):

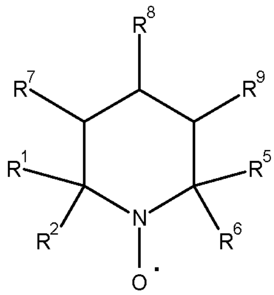

(V)

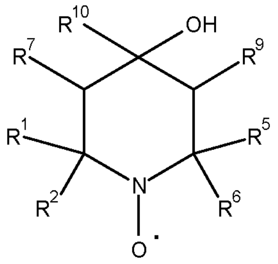

(VI)

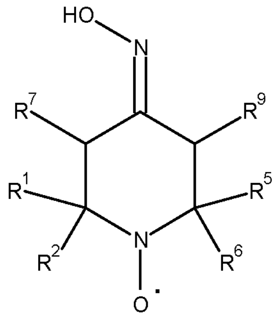

(VII)

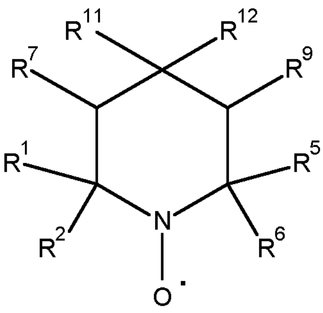

(VIII)

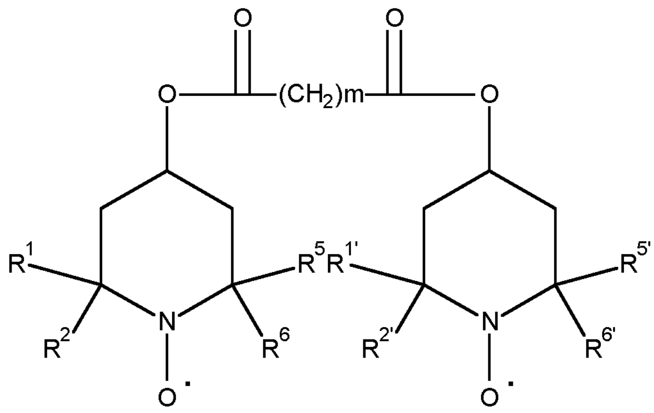

(IX)

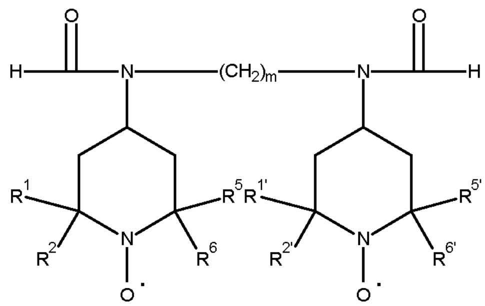

(X)

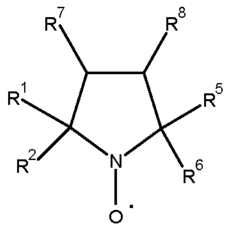

(XI)

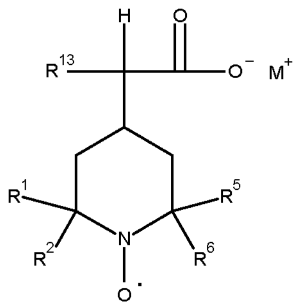

(XII)

mit

m =

2 bis 10,

$R^7$, $R^8$, $R^9$ =

unabhängig voneinander

H ,

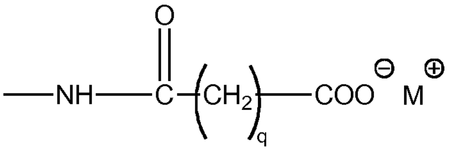
,
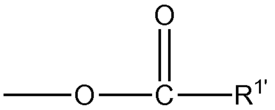
,
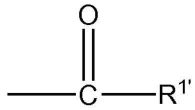
,

$-NH_2$,

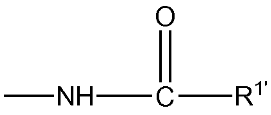 , 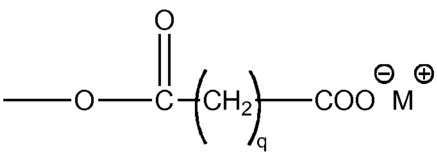 ,

$-COO^{\ominus}M^{\oplus}$, $-SO_3^{\ominus}M^{\oplus}$, $-PO_3^{\ominus}M^{\oplus}$,

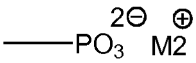 , 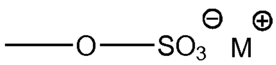 ,

-OH,

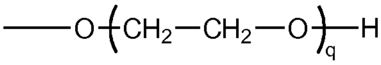

oder

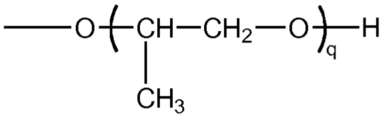 ,

$M^{\oplus}$=
ein Wasserstoff- oder ein Alkalimetallion,
q =
eine ganze Zahl von 1 bis 10,
$R^1$, $R^2$, $R^5$, $R^6$, =
unabhängig voneinander und unabhängig von $R^1$, $R^2$, $R^5$, $R^6$ dieselben Gruppen wie $R^1$,
$R^{10}$ =
$C_1$- bis $C_4$-Alkyl, $-CH{=}CH_2$, $-C{\equiv}CH$, -CN,

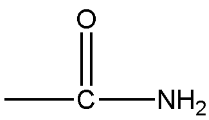 ,

$-COO^{\ominus}M^{\oplus}$, $-COOCH_3$ oder $COOC_2H_5$, $R^{11}$ =
ein organischer Rest, der wenigstens eine primäre, sekundäre (z.B. $-NHR^1$) oder tertiäre Aminogruppe (z.B. $-NR^1R^2$) oder wenigstens eine Ammoniumgruppe - $N^{\oplus}R^{14}R^{15}R^{16}X^{\ominus}$ aufweist, mit $X^{\ominus}$ = $F^{\ominus}$, $Cl^{\ominus}$, $Br^{\ominus}$, $HSO_4^{\ominus}$, $SO_4^{2\ominus}$, $H_2PO_4^{\ominus}$, $HPO_4^{2\ominus}$ oder $PO_4^{3\ominus}$ und $R^{14}$, $R^{15}$, $R^{16}$ voneinander unabhängige organische Reste (z.B. unabhängig voneinander und unabhängig von $R^1$ dieselben Gruppen wie $R^1$),
$R^{12}$ =
unabhängig von $R^{11}$ dieselben Gruppen wie $R^{11}$ oder -H, -OH, $C_1$- bis $C_4$-Alkyl, - $COO^{\ominus}M^{\oplus}$, $-C{\equiv}CH$,

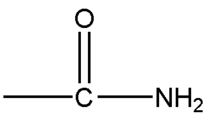 , 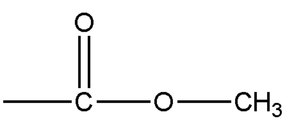 , 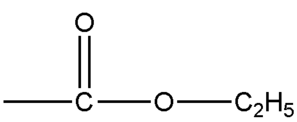 ,

oder hydroxysubstituiertes $C_1$- bis $C_4$-Alkyl (z.B. hydroxyethyl oder hydroxypropyl) oder $R^{11}$, $R^{12}$ = gemeinsam den Sauerstoff einer Carbonylgruppe und
$R^{13}$ = -H, $-CH_3$ oder

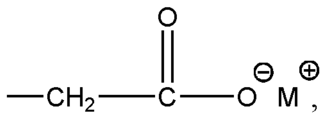

Vorzugsweise ist $R^1 = R^2 = R^5 = R^6 = R^{1'} = R^{2'} = R^{5'} = R^{6'} = -CH_3$.

Als beispielhafte Vertreter geeigneter N-Oxyl-Radikale seien 4-Hydroxy-2,2,6,6-tetramethyl-1-oxyl-piperidin, 4-Hydroxy-2,6-diphenyl-2,6-dimethyl-1-oxyl-piperidin, 4-Carboxy-2,2,6,6-tetramethyl-1-oxyl-piperidin, 4-Carboxy-2,6-diphenyl-2,6-dimethyl-1-oxyl-piperidin, 3-Carboxy-2,2,5,5-tetramethyl-1-oxyl-pyrrolidin, 3-Carboxy-2,5-diphenyl-2,5-dimethyl-1-oxyl-pyrrolidin, 4-Acetyl-2,2,6,6-tetramethyl-1-oxyl-piperidin, N,N'-Bis (1-Oxyl-2,2,6,6-tetramethylpiperidin-4-yl)-N,N'-bis formyl-1,6-diaminohexan und Bis-(1-oxyl-2,2,6,6-tetramethylpiperidin-4-yl)adipat genannt.

Die Herstellung von 3-Carboxy-2,2,5,5-tetramethyl-1-oxyl-pyrrolidin findet sich z.B. in Romanelli, M.; Ottaviani, M.F.; Martini, G.; Kevan, L., JPCH J: Phys. Chem., EN, 93, 1, 1989, S. 317-322.

Die Verbindungen (IX) und (X) können gemäß US-A 4665185 (z.B. Bsp. 7) sowie DE-A 19510184 erhalten werden.

Weitere geeignete beispielhafte Vertreter sind:

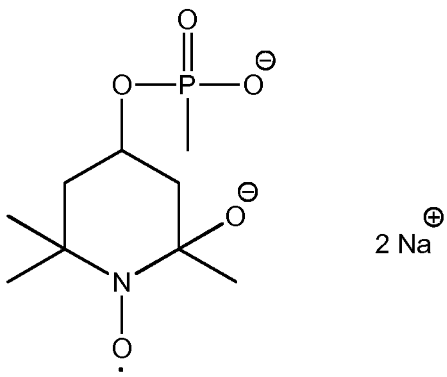

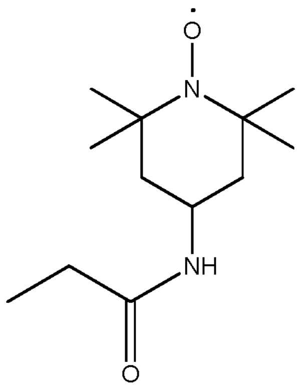

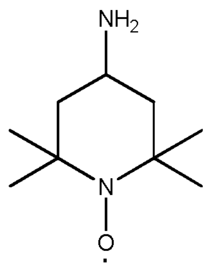

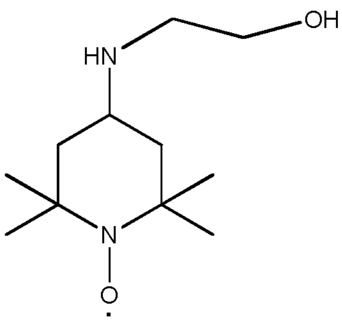

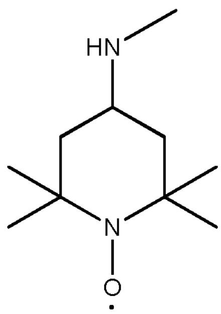

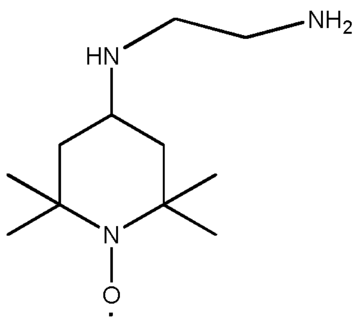

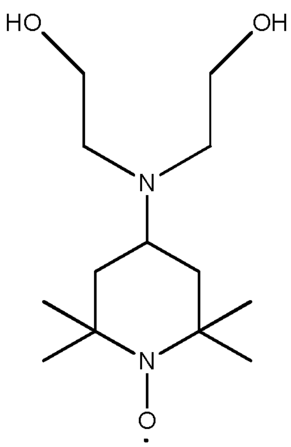

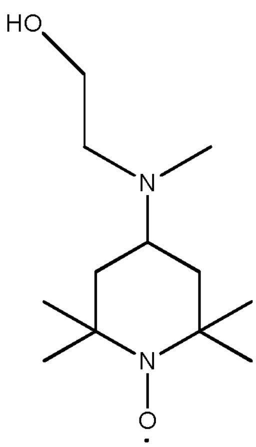

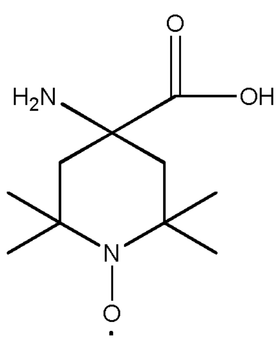

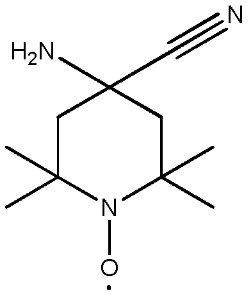
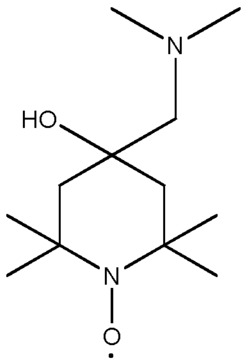
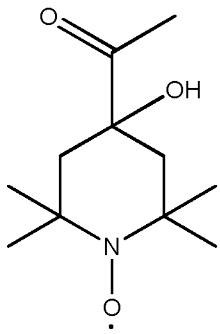
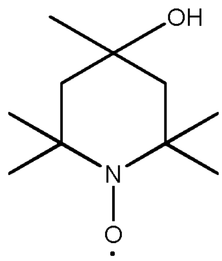

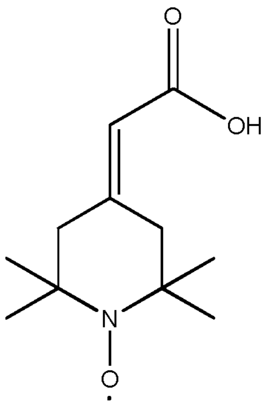
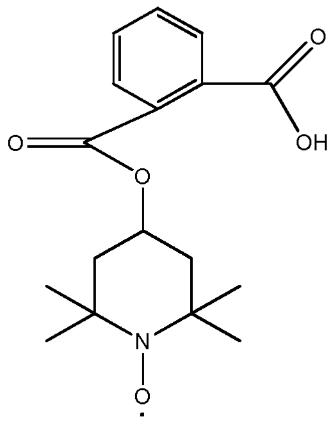
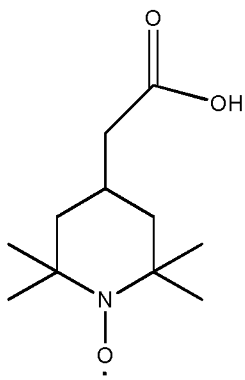

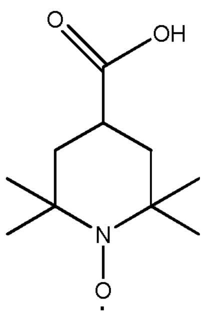
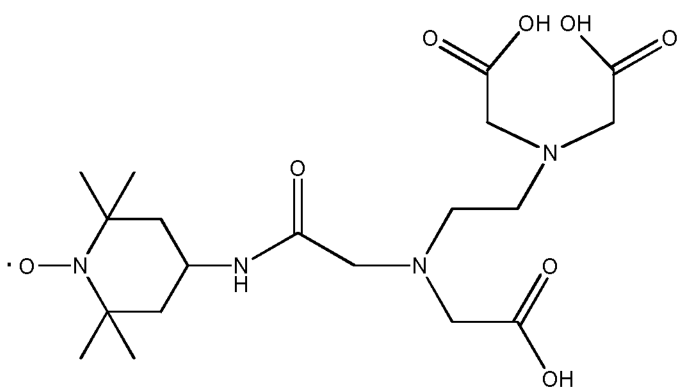

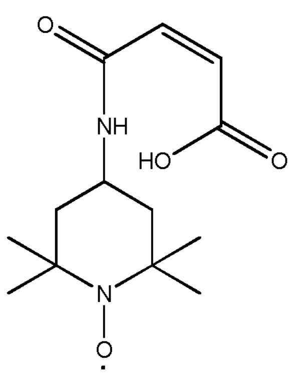
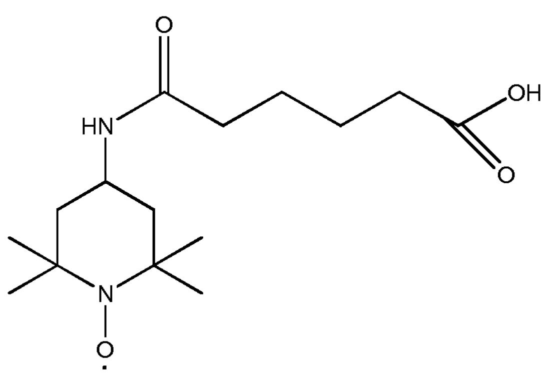

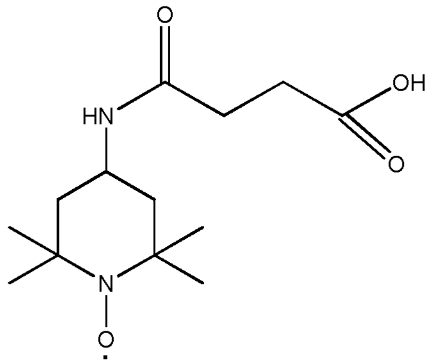

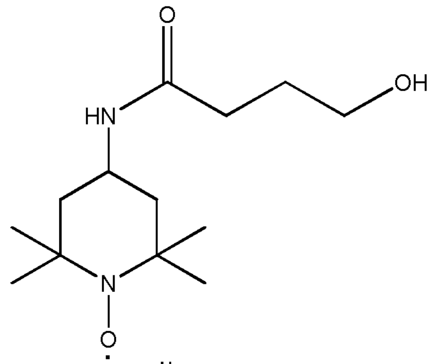

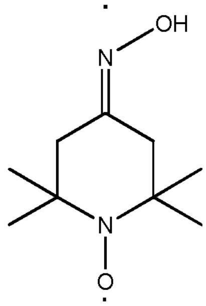

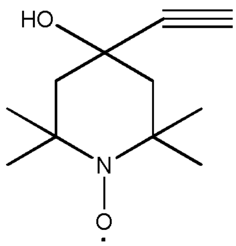

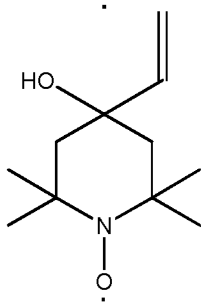

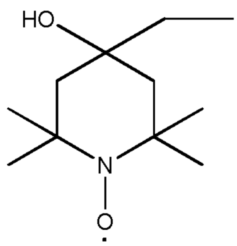

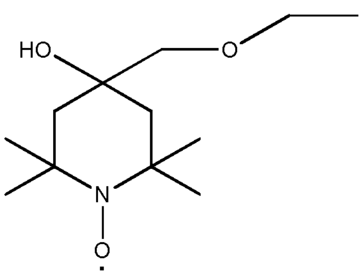

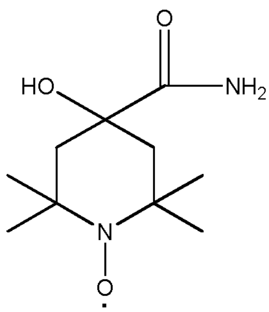

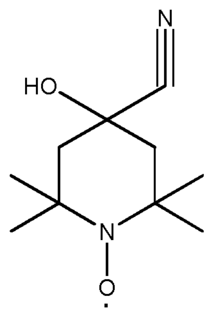

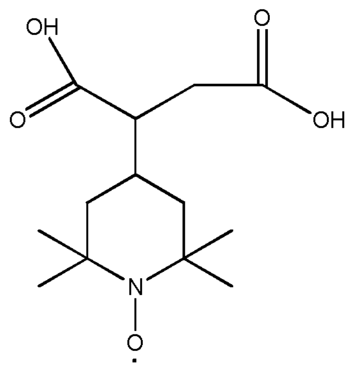

Geeignete organische Nitrosoverbindungen sind z.B. N-Nitrosoarylamine oder, die Nitrosogruppe unmittelbar an ein Kohlenstoffatom eines aromatischen Kerns gebunden aufweisende, Nitrosoverbindungen. Beispielhaft genannt seien Nitrosophenole wie 4-Nitrosophenol, Nitrosonaphthole wie 2-Nitroso-1-naphthol, Nitrosobenzol, N-Nitroso-N-methylharnstoff, Nitroso-N,N-Dialkylaniline mit Alkyl = Methyl, Ethyl, Propyl und/oder Butyl, N-Nitrosodiphenylamin, N-Nitrosophenylnaphthylamin, 4-Nitrosodinaphthylamin und p-Nitrosodiphenylamin.

Geeignete p-Phenylendiamine sind solche der allgemeine Formel (XIII)

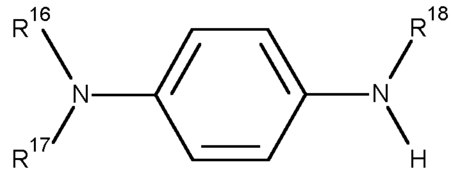

(XIII)

mit $R^{16}$, $R^{17}$, $R^{18}$= unabhängig voneinander Alkyl, Aryl, Alkaryl oder Aralkyl mit bis zu 20 C-Atomen, oder Wasserstoff.

Insbesondere eignen sich Verbindungen (XIII) mit $R^{16}$, $R^{17}$, $R^{18}$= unabhängig voneinander Methyl, Ethyl, Propyl, iso-Propyl, iso-Butyl, sek-Butyl, n-Butyl, Pentyl, Phenyl oder Naphthyl. Als Beispiele für geeignete Verbindungen XIII seien genannt: N,N'-Bis-sek-butyl-p-phenylendiamin, N-Phenyl-N'-isopropylphenylendiamin, N-Naphthyl-N'-sek-butyl-p-phenylendiamin, N,N,N'-Trimethyl-p-phenylendiamin, N,N,N'-Triethyl-p-phenylendiamin, N,N-Dimethyl-p-phenylendiamin, N,N-Diethyl-p-phenylendiamin, N-Phenyl-N',N'-dimethyl-p-phenylendiamin, N-Phenyl-N',N'-diethyl-p-phenylendiamin, N-Phenyl-N',N'-dipropyl-p-phenylendiamin, N-Phenyl-N',N'-di-nbutyl-p-phenylendiamin, N-Phenyl-N',N'-di-sek-butyl-p-phenylendiamin, N-Phenyl-N'-methyl-N'-ethyl-p-phenylendiamin, N-Phenyl-N'-methyl-N'propyl-p-phenylendiamin, N-Phenyl-N'-methyl-p-phenylendiamin, N-Phenyl-N'-ethyl-p-phenylendiamin, N-Phenyl-N'-propyl-p-phenylendiamin, N-Phenyl-N'-isopropyl-p-phenylendiamin, N-Phenyl-N'-butyl-p-phenylendiamin, N-Phenyl-N'-isobutyl-p-phenylendiamin, N-Phenyl-N'-sek-butyl-pphenylendiamin, N-Phenyl-N'-tert-butyl-p-phenylendiamin, N-Phenyl-N'-npentyl-p-phenylendiamin, N-Phenyl-N'-n-hexyl-p-phenylendiamin, N-Phenyl-N'-(1-methylhexyl)-p-phenylendiamin, N-Phenyl-N'-(1,3-dimethylbutyl)-p-phenylendiamin, N-Phenyl-N'-(1,4-dimethylpentyl)-p-phenylendiamin und p-Phenylendiamin.

Natürlich können auch Gemische aller verschiedenen vorgenannten Polymerisationsinhibitoren neben Phenothiazin eingesetzt werden.

Art und Menge dieser weiteren Bestandteile richten sich nach dem Verwendungszweck der Zusammensetzung.

Die erfindungsgemäße Zusammensetzung findet Verwendung bei der Stabilisierung von radikalisch polymerisierbaren Monomeren gegen radikalische Polymerisation.

Radikalisch polymerisierbare Monomere sind beispielsweise Vinylmonomere der allgemeinen Formel XIV

$$[CH_2=C(Y)-]_n-X \qquad (XIV),$$

worin X für ein Wasserstoffatom, ein Halogenatom, eine Carboxylgruppe, eine Sulfonsäuregruppe ($-SO_3H$), eine Phosphonsäuregruppe ($-PO_3H_2$), eine Silangruppe ($-SiH_3$) oder einen ein- bis zehnbindigen, vorzugsweise ein- bis sechsbindigen und bevorzugt ein- bis dreibindigen organischen oder metallorganischen Rest steht, und Y für ein Wasserstoffatom, ein Halogenatom, eine Nitrilgruppe, eine Alkylgruppe mit 1 bis 20 Kohlenstoffatomen, eine Cycloalkylgruppe mit 3 bis 20 Kohlenstoffatomen oder eine Arylgruppe mit 6 bis 22 Kohlenstoffatomen steht.

In der allgemeinen Formel XIV steht der Index n für eine ganze Zahl von 1 bis 10, vorzugsweise 1 bis 6 und bevorzugt 1 bis 3. Insbesondere ist n gleich 1.

Der Rest X steht für ein Wasserstoffatom, ein Halogenatom, eine Carboxylgruppe, eine Sulfonsäuregruppe ($-SO_3H$), eine Phosphonsäuregruppe ($-PO_3H_2$), eine Silangruppe ($-SiH_3$) oder einen ein- bis zehnbindigen, vorzugsweise ein- bis sechsbindigen und bevorzugt ein- bis dreibindigen organischen oder metallorganischen Rest. Insbesondere handelt es sich um einen einbindigen organischen Rest.

Unter einem organischen Rest wird ein Rest verstanden, der mindestens ein Kohlenstoffatom enthält.

Unter einem metallorganischen Rest wird ein Rest verstanden, der mindestens ein Kohlenstoffatom sowie mindestens ein Siliziumatom und/oder mindestens ein Boratom, insbesondere mindestens ein Siliziumatom, enthält.

Der organische und der metallorganische Rest können niedermolekular, oligomer oder polymer sein. »Niedermolekular« bedeutet, dass der betreffende Rest aus einer Struktureinheit oder zwei gleichen oder verschiedenen Struktureinheiten aufgebaut ist. »Oligomer« bedeutet, dass der betreffende Rest aus 2 bis 12 gleichen oder verschiedenen Struktureinheiten aufgebaut ist. »Polymer« bedeutet, dass der betreffende Rest aus mehr als 12 gleichen oder verschiedenen Struktureinheiten aufgebaut ist.

Die Struktureinheiten des organischen und des metallorganischen Restes können mindestens ein Heteroatom, vorzugsweise ausgewählt aus der Gruppe, bestehend aus Sauerstoff, Schwefel, Stickstoff, Phosphor, Fluor, Chlor und Brom, bevorzugt Sauerstoff, Schwefel und Phosphor, insbesondere Sauerstoff, enthalten.

Besonders bevorzugt sind die Vinylmonomeren der allgemeinen Formel XIV, worin n für 1 und X für ein Wasserstoffatom, ein Halogenatom, eine Sulfonsäuregruppe, eine Phosphonsäuregruppe, eine Silangruppe ($-SiH_3$) oder einen einbindigen organischen oder metallorganischen Rest stehen. Solche Monomere werden auch als Vinylmonomere im engeren Sinne bezeichnet (vgl. a. Römpp Online 2007, »Vinylmonomere«).

Beispiele gut geeigneter Halogenatome X sind Fluor, Chlor und Brom, insbesondere Chlor.

Beispiele gut geeigneter einbindiger organischer Reste X sind Alkylreste R vorzugsweise mit 1 bis 12, bevorzugt

1 bis 10 und insbesondere 1 bis 8 Kohlenstoffatomen, Cycloalkylreste R vorzugsweise mit 3 bis 10, bevorzugt 4 bis 8 und insbesondere 5 oder 6 Kohlenstoffatomen, Arylreste R vorzugsweise mit 6 bis 22, bevorzugt 6 bis 16 und insbesondere 6 bis 10 Kohlenstoffatomen, Alkyl-, Cycloalkyl- und Aryletherreste (-OR), worin der Rest R vorzugsweise aus der Gruppe, bestehend aus den vorstehend genannten Alkylresten, Cycloalkylresten und Arylresten R, ausgewählt ist, Nitrilgruppe (-CN), Carboxylgruppe (-COOH), Carboxylalkylester-Reste (-O-CO-R oder -CO-O-R) vorzugsweise mit 1 bis 10, bevorzugt 1 bis 8 und insbesondere 1 bis 6 Kohlenstoffatomen im Alkylrest R, Carboxylcycloalkylester-Reste (-O-CO-R oder -CO-O-R) vorzugsweise mit 3 bis 10, bevorzugt 4 bis 8 und insbesondere 5 bis 6 Kohlenstoffatomen im Cycloalkylrest R, Carboxylarylester-Reste (-O-CO-R oder -CO-O-R) vorzugsweise mit 6 bis 22, bevorzugt 6 bis 16 und insbesondere 6 bis 10 Kohlenstoffatomen im Arylrest R, Carboxylamid-Rest ($-CO-NH_2$), Carboxylamid-Reste (-CO-NRH oder $-CO-NR_2$), die am Stickstoff mit mindestens einem Rest R substituiert sind, der vorzugsweise aus der Gruppe, bestehend aus den vorstehend genannten Alkylresten, Cycloalkylresten und Arylresten R, wobei zwei Reste R auch cyclisch miteinander verknüpft sein können, ausgewählt ist, Carboxylamid-Reste (-NR-CO-R), worin der Rest R vorzugsweise aus der Gruppe, bestehend aus den vorstehend genannten Alkylresten, Cycloalkylresten und Arylresten R sowie Wasserstoffatom, ausgewählt ist oder worin die beiden Reste R cyclisch miteinander verknüpft sind, so dass vorzugsweise ein vier-, fünf- oder sechsgliedriger Ring resultiert, und Aminoreste (-NHR oder $-NR_2$), die am Stickstoffatom mit mindestens einem Rest substituiert sind, der vorzugsweise aus der Gruppe, bestehend aus den vorstehend genannten Alkylresten, Cycloalkylresten und Arylresten R, wobei zwei Reste R auch cyclisch miteinander verknüpft sein können, ausgewählt ist, wobei die Reste R substituiert oder unsubstituiert sein können.

Beispiele gut geeigneter Substituenten für die substituierten Reste R sind Halogenatome, bevorzugt Fluor, Chlor und Brom, insbesondere Fluor und Chlor, Nitrilgruppen, Nitrogruppen, Carboxylgruppen, Sulfonsäuregruppen, Etherreste (-OR), Esterreste (-O-CO-R oder -CO-O-R), Carboxylamid-Reste (-NH-CO-R) und Aminoreste (-NHR oder-$NR_2$), insbesondere Carboxylgruppen und Sulfonsäuregruppen. Hierin haben die Reste R die vorstehend angegebene Bedeutung.

Beispiele gut geeigneter einbindiger metallorganischer Reste X sind Silylreste ($-SiH_2R$, $-SiHR_2$ oder $-SiR_3$), worin der Rest R vorzugsweise aus der Gruppe, bestehend aus den vorstehend genannten Alkylresten, Cycloalkylresten und Arylresten R, wobei 2 oder 3 Reste R auch cyclisch miteinander verknüpft sein können, ausgewählt ist und Silyletherreste ($-SiH_2(OR)$, $-SiH(OR)_2$, $-Si(OR)_3$, -SiHR(OR), $-SiR_2(OR)$, oder-$SiR(OR)_2$), worin der Rest R vorzugsweise aus der Gruppe, bestehend aus den vorstehend genannten Alkylresten R, Cycloalkylresten und Arylresten R, wobei 2 oder 3 Reste R auch cyclisch miteinander verknüpft sein können, ausgewählt ist, wobei diese Silylreste und Silyletherreste X auch über ein Sauerstoffatom mit der Vinylgruppe verbunden sein können.

In der allgemeinen Formel XIV steht die Variable Y für ein Wasserstoffatom, ein Halogenatom, eine Nitrilgruppe, eine Alkylgruppe mit 1 bis 20 Kohlenstoffatomen, eine Cycloalkylgruppe mit 3 bis 20 Kohlenstoffatomen oder eine Arylgruppe mit 6 bis 22 Kohlenstoffatomen, bevorzugt für ein Wasserstoffatom, ein Halogenatom, eine Nitrilgruppe, eine Methylgruppe oder Ethylgruppe, insbesondere für ein Wasserstoffatom oder eine Methylgruppe.

Gut geeignete Vinylmonomere XIV entstammen den Verbindungsklassen der 1-Olefine, Vinylhalogenide, Vinylaromaten, Vinylether, Vinylester, Vinylamide, Vinylsilane, Vinylsilylether, Vinylsiloxane, Vinylamine, Vinylamide, Vinylsulfonsäure, Vinylphosphonsäure, Vinylcarbonsäuren, Vinylaromatsulfonsäuren, Acrylsäuren, Acrylsäureester, Acrylamide, Acrylnitrile, Allylether und Allylester.

Besonders gut geeignete Vinylmonomere XIV entstammen den Verbindungsklassen der 1-Olefine, Vinylhalogenide, Vinylaromaten, Vinylether, Vinylester, Vinylamide, Vinylsulfonsäure, Vinylphosphonsäure, Vinylcarbonsäuren, Vinylaromatsulfonsäuren, Acrylsäuren, Acrylsäureester, Acrylamide und Acrylnitrile.

Beispiele besonders gut geeigneter 1-Olefine sind Ethylen, Propen, 1-Buten, 1-Penten und 1-Hexen.

Beispiele besonders gut geeigneter Vinylhalogenide sind Vinylfluorid, Vinylchlorid und Vinylbromid.

Beispiele besonders gut geeigneter Vinylaromaten sind Styrol und alpha-Methylstyrol.

Beispiele besonders gut geeigneter Vinylether sind Vinylmethyl-, Vinylethyl-, Vinylpropyl- und Vinylbutylether.

Beispiele besonders gut geeigneter Vinylester sind Vinylacetat und Vinylpropionat.

Beispiele besonders gut geeigneter Vinylamide sind N-Vinylformamid, N-Vinylacetamid, N-Vinylpyrrolidon und N-Vinylcaprolactam.

Ein Beispiel für eine besonders gut geeignete Vinylcarbonsäuren ist Vinylessigsäure.

Beispiele besonders gut geeigneter Vinylaromatsulfonsäuren sind Vinylbenzol-2-, -3- und -4-sulfonsäure.

Beispiele besonders gut geeigneter Acrylsäuren sind Acrylsäure, Methacrylsäure, Ethacrylsäure, Chloracrylsäure und Cyanacrylsäure, insbesondere Acrylsäure und Methacrylsäure.

Beispiele besonders gut geeigneter Acrylsäureester sind Acrylsäure- und Methacrylsäuremethylester, -ethylester, -propylester, -i-propylester, -n-butylester, -i-butylester-1,1-diethylbutylester, -2-ethylhexylester, -1,1-diethylpropylester, -1-methyl-1-ethylpropylester, -1-methyl-1-ethylbutylester, -1,1-dimethylbutylester, -1,1-dimethylpropylester und -tert.-butylester, bevorzugt Acrylsäuremethylester, Acrylsäureethylester, Acrylsäure-n-butylester oder n-Butylacrylat (NBA), Acrylsäure-isobutylester oder iso-Butylacrylat (IBA), Acrylsäure-tert-butylester oder tert-Butylacrylat, Acrylsäure-2-ethylhexylester oder 2-Ethylhexylacrylat (EHA), Hydroxyalkylacrylsäureester, wie z.B. Acrylsäure-(2-hydroxyethyl)ester, Acrylsäure-(2-hydroxypropyl)ester, Acrylsäure-(4-hydroxybutyl)ester und Methacrylsäure-(2-hydroxyethyl)ester und Methacrylsäure-(2-hydroxypropyl)ester, tert-Butylmethacrylat (TBMA) und 2-Ethylhexylmethacrylat (MEHA).

Beispiele besonders gut geeigneter Acrylamide sind Acrylamid und Methacrylamid.

Beispiele besonders gut geeigneter Acrylnitrile sind Acrylnitril und Methacrylnitril.

Weiterhin betrifft die Erfindung ein Verfahren zur Sofortbeendigung von radikalischen Polymerisationen, wobei man die erfindungsgemäße Zusammensetzung einem radikalisch polymerisierenden System zusetzt.

Die erfindungsgemäße Zusammensetzung findet vorzugsweise Verwendung in einem Verfahren zur Inhibierung einer unkontrollierten radikalischen Polymerisation radikalisch polymerisierbarer Monomere in einem Behältnis. Der Beginn einer unkontrollierten radikalischen Polymerisation kann durch die Detektion eines Temperaturanstiegs im Behältnis oder durch Detektion eines besonders steilen Temperaturanstiegsgradienten (wenn der Temperaturanstieg des Systems pro Zeit über einem vorgegebenen Wert liegt) registriert werden. Wird der Beginn einer unkontrollierten radikalischen Polymerisation detektiert, wird die erfindungsgemäße Zusammensetzung in das Behältnis eingebracht und eingemischt.

Das Behältnis kann dabei jedes Gefäß oder jeder Behälter sein, der dafür geeignet ist radikalisch polymerisierbare Monomere zu beinhalten, z.B. Reaktionsgefäße, wie Reaktoren, oder Lagerbehälter zur Aufbewahrung. Insbesondere von Bedeutung ist das genannte Verfahren zur Verhinderung der unkontrollierten Polymerisation von Acrylsäure in einem Reaktor oder Lagerbehälter.

Im Rahmen der Prozessüberwachung kann eine beginnende Durchgehreaktion von Monomeren im Behältnis detektiert werden. Die Detektion kann zum einen durch Überschreitung von gestaffelten Temperaturschwellen erfolgen, zum anderen durch die Unterschreitung eines Zeitlimits für die Zeit eines Temperaturanstiegs zwischen zwei Temperaturschwellen. Die Temperaturschwellen und die Zeiten werden für die angestrebte Verwendung zweckmäßig gewählt. Der effektivste Weg die beginnende Durchgehreaktion zu terminieren, ist die Zugabe von Inhibitoren der radikalischen Polymerisation. Die Inhibitoren der radikalischen Polymerisation werden dabei erfindungsgemäß als Bestandteil der oben definierten erfindungsgemäßen Zusammensetzung zugegeben. Insbesondere erfolgt die Zugabe der Zusammensetzung durch Injektion in das Behältnis, in dem in einem ersten Schritt der Notfallerkennung bereits der Temperaturanstieg detektiert wurde. Die erfindungsgemäße Zusammensetzung wird dabei in einer Menge zugegeben, die ausreicht, um die beginnende, unkontrollierte radikalische Polymerisation zu stoppen. Üblicherweise wird eine solche Menge der erfindungsgemäßen Zusammensetzung zugegeben, dass eine Endkonzentration des Inhibitors von 50 bis 1000 ppm erreicht wird. Bevorzugt wird eine solche Menge der erfindungsgemäßen Zusammensetzung zugegeben, dass eine Endkonzentration des Inhibitors von 100 bis 500 ppm erreicht wird und besonders bevorzugt eine solche Menge der erfindungsgemäßen Zusammensetzung, dass eine Endkonzentration des Inhibitors von 125 bis 250 ppm erreicht wird.

Die Erfindung wird durch die beigefügten Figuren und Beispiele näher erläutert.

**Figur 1** ist ein Balkendiagramm, das die relative Löslichkeit von Phenothiazin in einem Gemisch von EMIM-Isononanat und Lösungsmittel (Gewichtsverhältnis 20/80), bezogen auf die Löslichkeit von Phenothiazin im reinen Lösungsmittel, zeigt.

**Figur 2** ist ein Balkendiagramm, das die relative Löslichkeit von Phenothiazin in einem Gemisch von EMIM-Ethylsulfat und Lösungsmittel (Gewichtsverhältnis 20/80), bezogen auf die Löslichkeit von Phenothiazin im reinen Lösungsmittel, zeigt.

**Figur 3** ist ein Balkendiagramm, das die relative Löslichkeit von Phenothiazin in einem Gemisch von TBA-Ethercarboxylat und Lösungsmittel (Gewichtsverhältnis 20/80), bezogen auf die Löslichkeit von Phenothiazin im reinen Lösungsmittel, zeigt.

**Figur 4** ist ein Balkendiagramm, das die relative Löslichkeit von Phenothiazin in einem Gemisch von EMIM-Acetat und Lösungsmittel (Gewichtsverhältnis 20/80), bezogen auf die Löslichkeit von Phenothiazin im reinen Lösungsmittel, zeigt.

**Figur 5** zeigt das Phasendiagramm (Dreiecksdiagramm) des Systems Phenothiazin/Proglyme/EMIM Acetat.

**Figur 6** zeigt das Phasendiagramm (Dreiecksdiagramm) des Systems Phenothiazin/Proglyme/EMIM Ethylsulfat.

Beispiel 1

In diesem Beispiel wurde das Kristallisationsverhalten einer Lösung von Phenothiazin in einem Gemisch von ionischer Flüssigkeit und Lösungsmittel (Gewichtsverhältnis ionische Flüssigkeit/Lösungsmittel = 20/80) untersucht. Die

Zusammensetzungen wurden über 14 Tage jeweils tagsüber bei -70°C (Trockeneis) und über Nacht bei -20°C gelagert und anschließend bei Raumtemperatur optisch beurteilt.

Es sind die Proben als gut zu bewerten, die nach 14 Tagen keinen Bodensatz oder Kristalle zeigen. Die nachfolgenden Tabellen zeigen die Zusammensetzungen und die Ergebnisse für Phenothiazin in 4 verschiedene ionische Flüssigkeiten kombiniert mit unterschiedlichen Lösungsmitteln.

Tabelle 2: Kristallisationstests für mit PTZ gesättigte EMIM Acetat/Lösungsmittelmischungen (20/80)

| Lösungsmittel | EMIM Acetat [%] | PTZ [%] | Beurteilung nach 14 Tagen |
|---|---|---|---|
| DMSO | 8,9 | 55,6 | Flüssig |
| Benzonitril | 12,5 | 37,5 | Flüssig + Bodensatz |
| Dimethylsuccinat | 12,9 | 35,5 | Flüssig + Bodensatz |
| Methylbenzoat | 12,5 | 37,5 | Kristalliner Bodensatz, flüssig |
| Acetonitril | 12,9 | 35,5 | Flüssig + Kristalle |
| Methylbutylketon | 11,8 | 41,2 | Flüssig + Bodensatz |
| Ethylendiamin | 13,3 | 33,3 | Flüssig + Kristalle |
| N-Methylimidazol | 9,3 | 53,5 | Kristallbrei |
| Proglyme | 12,5 | 37,5 | Flüssig |

Tabelle 3: Kristallisationstests für mit PTZ gesättigte EMIM Isononanat/Lösungsmittelmischungen (20/80)

| Lösungsmittel | EMIM Isononanat [%] | PTZ [%] | Beurteilung nach 14 Tagen |
|---|---|---|---|
| DMSO | 9,3 | 53,5 | Flüssig |
| Methylbutylketon | 13,3 | 33,3 | Flüssig + Bodensatz |
| Ethylendiamin | 13,3 | 33,3 | Flüssig + Bodensatz |
| N-Methylimidazol | 11,1 | 44,4 | Flüssig |
| N-Methylimidazol | 10,0 | 50,0 | Flüssig |
| Proglyme | 13,8 | 31,0 | Flüssig |

Tabelle 4: Kristallisationstests für mit PTZ gesättigte EMIM Ethylsulfat/Lösungsmittelmischungen (20/80)

| Lösungsmittel | EMIM Ethylsulfat [%] | PTZ [%] | Beurteilung nach 14 Tagen |
|---|---|---|---|
| Acetonitril | 12,1 | 39,4 | Weißer Bodensatz, flüssig |
| Methylbutylketon | 13,8 | 31,0 | Gelber Bodensatz, flüssig |
| N-Methylimidazol | 10,8 | 45,9 | Kristalle, flüssig |
| N-Methylimidazol | 12,0 | 40,1 | Flüssig |
| N-Methylimidazol | 11,0 | 45,1 | Flüssig |
| Proglyme | 14,3 | 28,6 | Flüssig |

Tabelle 5: Kristallisationstests für mit PTZ gesättigte TBA Ethercarboxylat/Lösungsmittelmischungen (20/80)

| Lösungsmittel | TBA Ethercarboxylat [%] | PTZ [%] | Beurteilung nach 14 Tagen |
|---|---|---|---|
| Methylbutylketon | 14,3 | 28,6 | Weißer Bodensatz, flüssig |
| N-Methylimidazol | 12,0 | 40,1 | Flüssig |
| N-Methylimidazol | 11,0 | 45,1 | Flüssig |

(fortgesetzt)

| Lösungsmittel | TBA Ethercarboxylat [%] | PTZ [%] | Beurteilung nach 14 Tagen |
|---|---|---|---|
| Dimethylethylenharnstoff | 12,0 | 40,1 | Kristalliner Bodensatz, flüssig |
| Dimethylethylenharnstoff | 11,0 | 45,1 | Kristalle, flüssig |
| Proglyme | 14,3 | 28,6 | Flüssig |

Beispiel 2

In diesem Beispiel wurden Tempertests in Acrylsäure (AA) durchgeführt. Bei diesem Test wird eine Probe von Acrylsäure bei 120 °C unter Luftatmosphäre gehalten und die Zeitspanne bis zum Auftreten einer Trübung der Lösung bestimmt. Die Zeitspanne wird als die Inhibierungsperiode (IP) der Acrylsäure-Probe betrachtet.

1. Herstellung von mit unterschiedlichen Polymerisationsinhibitoren versetzten flüssigen Phasen.

Frisch hergestellte Reinacrylsäure (GAA, Herstellung wie in der DE-A 102007055086 beschrieben), die, bezogen auf ihr Gewicht, mit 200 Gew.-ppm Methoxyphenol (MEHQ) polymerisationsinhibiert war, wurde unter vermindertem Druck (1000 Pa) durch zweifaches, aufeinanderfolgend durchgeführtes Überdestillieren von MEHQ befreit. Die Reinheit des so erzeugten Reinacrylsäuredestillats RD betrug > 99,8 Gew.-%, bei einem Aldehyd- und Ketongesamtgehalt < 5 Gew.-ppm, einem Diacrylsäuregehalt < 1 Gew.-ppm und einem Propionsäuregehalt von < 200 Gew.-ppm.

Aus dem Reinacrylsäuredestillat RD wurde eine Teilmenge 1 entnommen und unter Rühren eine Stammlösung 1 erzeugt, die 1000 Gew.-ppm Phenothiazin (PTZ) enthielt. Aus einer anderen Teilmenge 2 des Reinacrylsäuredestillates RD wurden mit unterschiedlichen Mengen an Ionischen Flüssigkeiten IL, Lösungsmitteln LM oder Mischungen IL/LM von Ionischer Flüssigkeit IL mit Lösungsmittel LM verschiedene Stammlösungen 2 erzeugt, in welchen z.B. unterschiedliche Mengen verschiedener Ionischer Flüssigkeiten IL, verschiedener Lösungsmittel LM oder verschiedene Mischungen IL/LM von Ionischer Flüssigkeit IL mit Lösungsmittel LM gelöst waren.

Von der Stammlösung 1 wurden 40 Gew.-Teile mit 960 Gew.-Teilen des Reinacrylsäuredestillates RD vermischt und so die Stammlösung 3 erhalten. Aus dieser Stammlösung 3 wurde eine Untermenge in identische Proben mit einem Volumen von 1 mL aufgeteilt.

Aus den Stammlösungen 2 entnommene Probemengen wurden mit den aus den Stammlösungen 3 erhaltenen verschiedenen 1 mL Proben so vereinigt, dass die gewünschten Zusammensetzungen an Acrylsäure, Phenothiazin (PTZ), Ionischen Flüssigkeiten IL, Lösungsmitteln LM sowie Mischungen IL/LM von Ionischen Flüssigkeiten IL mit Lösungsmitteln LM durch Dotierung erhalten wurden. Die so erzeugten dotierten Proben wurden für die weitere Untersuchung am selben Tage bereitgestellt. Für Wiederholungsmessungen wurden neue dotierte Proben erzeugt, um den Einfluss der durch Michael-Oligomerisierung erhaltenen Acrylsäure-Oligomere zu minimieren.

2. Untersuchung der Polymerisationsneigung der dotierten Proben der verschiedenen flüssigen Phasen P.

Zur Untersuchung der Polymerisationsneigung der jeweiligen dotierten Probe wurden jeweils drei HPLC-Vials (transparentes Gefäße mit 1,5 mL Füllvolumen) mit je 0,5 mL der jeweiligen Probe unter Luft befüllt und anschließend mit einer Bördelkappe dicht verschlossen. Unmittelbar nach Fertigstellung wurden jeweils bis zu 92 wie beschrieben befüllte Vials in eine dafür angefertigte Halterung eingehängt und bei einer Temperatur von 120°C in einem Umlufttrockenschrank sich selbst überlassen, während die Halterung mit sechs Umdrehungen pro Minute rotierte, um eine vollständige Durchmischung in den Vials zu gewährleisten (sechsmal pro Minute kam der flüssige Inhalt des jeweiligen Vials mit der Bördelkappe in Berührung). Dann wurde die Zeit T bis zur vollständigen Polymerisation der jeweiligen Probe im zugehörigen Vial erfasst. Dazu wurden die Proben in den Vials im Trockenschrank mit Hilfe einer digitalen Videokamera überwacht und der Videofilm nachträglich visuell ausgewertet.

Für jede dotierte Probe wurden so drei zugehörige Werte für T ermittelt, die arithmetisch gemittelt wurden. Die resultierenden Mittelwerte IP (in Minuten) für die verschiedenen Proben, einschließlich ihrer zugehörigen relevanten Gehalte an von Acrylsäure verschiedenen Bestandteilen, sind nachfolgend aufgelistet (die Gehaltsangaben sind jeweils bezogen auf die in der jeweiligen Probe enthaltenen Gesamtmasse).

Wurden Mischungen von ionischer Flüssigkeit und Lösungsmittel als Additiv eingesetzt, so war das Gewichtsverhältnis von Ionischer Flüssigkeit zu Lösungsmittel bzw. zum Lösungsmittelgemisch (IL/LM) gleich 20/80.

Tabelle 6 Inhibierungsperioden für stabilisierte Mischungen aus AA mit Phenothiazin und Lösungsmittel oder Phenothiazin und ionischer Flüssigkeit oder Phenothiazin und ionischer Flüssigkeit und Lösungsmittel.

| Additiv | | Inhibitor | | Inhibierungsperiode |
|---|---|---|---|---|
| LM, IL oder IL/LM | Konzentration | Verbindung | Konzentration | |
| | ppm | | ppm | min |
| | | | | |
| - | - | - | - | 240 min |
| DMSO | 1000 ppm | PTZ | 20 ppm | 317 min |
| Sulfolan | 1000 ppm | PTZ | 20 ppm | 330 min |
| VE-Wasser | 1000 ppm | PTZ | 20 ppm | 327 min |
| Benzonitril | 1000 ppm | PTZ | 20 ppm | 334 min |
| Essigsäure | 1000 ppm | PTZ | 20 ppm | 347 min |
| Propionsäure | 1000 ppm | PTZ | 20 ppm | 337 min |
| Dimethylsuccinat | 1000 ppm | PTZ | 20 ppm | 348 min |
| Methylbenzoat | 1000 ppm | PTZ | 20 ppm | 350 min |
| g-Butyrolacton | 1000 ppm | PTZ | 20 ppm | 361 min |
| Acetonitril | 1000 ppm | PTZ | 20 ppm | 335 min |
| Cyclohexanon | 1000 ppm | PTZ | 20 ppm | 75 min |
| Methylbutylketon | 1000 ppm | PTZ | 20 ppm | 309 min |
| Morpholin | 1000 ppm | PTZ | 20 ppm | 410 min |
| Ethylenglycol | 1000 ppm | PTZ | 20 ppm | 365 min |
| Pluriol A 500 E | 1000 ppm | PTZ | 20 ppm | 320 min |
| Pluriol E 400 | 1000 ppm | PTZ | 20 ppm | 292 min |
| Agnique AMD 3 L | 1000 ppm | PTZ | 20 ppm | 405 min |
| Cetiol B | 1000 ppm | PTZ | 20 ppm | 347 min |
| Plastomoll DOA | 1000 ppm | PTZ | 20 ppm | 350 min |
| Agnique FOH 898 | 1000 ppm | PTZ | 20 ppm | 332 min |
| Agnique AMD 10 | 1000 ppm | PTZ | 20 ppm | 335 min |
| Ethylendiamin | 1000 ppm | PTZ | 20 ppm | 341 min |
| 3-(Dimethylamino)-1-propylamin | 1000 ppm | PTZ | 20 ppm | 407 min |
| N-Methylimidazol | 1000 ppm | PTZ | 20 ppm | 366 min |
| Dimethylethylenharnstoff | 1000 ppm | PTZ | 20 ppm | 313 min |
| Agnique AE 3 2 EH | 1000 ppm | PTZ | 20 ppm | 279 min |
| Benzylalkohol | 1000 ppm | PTZ | 20 ppm | 355 min |
| Dimethylphthalat | 1000 ppm | PTZ | 20 ppm | 348 min |
| Dowtherm A | 1000 ppm | PTZ | 20 ppm | 323 min |
| Anisacetal | 1000 ppm | PTZ | 20 ppm | 320 min |
| Decahydronaphthalen | 1000 ppm | PTZ | 20 ppm | 347 min |
| Agnique FOH 9 OC | 1000 ppm | PTZ | 20 ppm | 255 min |
| Proglyme | 1000 ppm | PTZ | 20 ppm | 339 min |

(fortgesetzt)

| Additiv | | Inhibitor | | Inhibierungsperiode |
|---|---|---|---|---|
| LM, IL oder IL/LM | Konzentration | Verbindung | Konzentration | |
| | ppm | | ppm | min |
| NMP | 1000 ppm | PTZ | 20 ppm | 343 min |
| EMIM - Acetat | 1000 ppm | PTZ | 20 ppm | 296 min |
| EMIM - Acetat / DMSO | 1000 ppm | PTZ | 20 ppm | 378 min |
| EMIM - Acetat / Benzonitril | 1000 ppm | PTZ | 20 ppm | 416 min |
| EMIM - Acetat / Dimethylsuccinat | 36 ppm | PTZ | 20 ppm | 372 min |
| EMIM - Acetat / gamma-Butyrolacton | 1000 ppm | PTZ | 20 ppm | 414 min |
| EMIM - Acetat / Acetonitril | 1000 ppm | PTZ | 20 ppm | 411 min |
| EMIM - Acetat / Methylbutylketon | 29 ppm | PTZ | 20 ppm | 351 min |
| EMIM - Acetat / Ethylendiamin | 1000 ppm | PTZ | 20 ppm | 356 min |
| EMIM - Acetat / 3-(Dimethylamino)-1-propylamin | 25 ppm | PTZ | 20 ppm | 422 min |
| EMIM - Acetat / N-Methylimidazol | 1000 ppm | PTZ | 20 ppm | 451 min |
| EMIM - Acetat / Proglyme | 33 ppm | PTZ | 20 ppm | 395 min |
| EMIM - Isononanoat | 1000 ppm | PTZ | 20 ppm | 379 min |
| EMIM - Isononanoat / DMSO | 1000 ppm | PTZ | 20 ppm | 373 min |
| EMIM - Isononanoat / g-Butyrolacton | 33 ppm | PTZ | 20 ppm | 368 min |
| EMIM - Isononanoat / Methylbutylketon | 40 ppm | PTZ | 20 ppm | 382 min |
| EMIM - Isononanoat / Ethylendiamin | 1000 ppm | PTZ | 20 ppm | 416 min |
| EMIM - Isononanoat / 3-(Dimethylamino)-1-propylamin | 1000 ppm | PTZ | 20 ppm | 575 min |
| EMIM - Isononanoat / N-Methylimidazol | 1000 ppm | PTZ | 20 ppm | 469 min |
| EMIM - Isononanoat / Proglyme | 44 ppm | PTZ | 20 ppm | 333 min |
| EMIM - Ethylsulfat | 29 ppm | PTZ | 20 ppm | 386 min |
| EMIM - Ethylsulfat / gamma-Butyrolacton | 1000 ppm | PTZ | 20 ppm | 361 min |
| EMIM - Ethylsulfat / Acetonitril | 1000 ppm | PTZ | 20 ppm | 350 min |
| EMIM - Ethylsulfat / Methylbutylketon | 44 ppm | PTZ | 20 ppm | 329 min |
| EMIM - Ethylsulfat / Ethylendiamin | 25 ppm | PTZ | 20 ppm | 362 min |
| EMIM - Ethylsulfat / 3-(Dimethylamino)-1-propylamin | 29 ppm | PTZ | 20 ppm | 372 min |
| EMIM - Ethylsulfat / N-Methylimidazol | 1000 ppm | PTZ | 20 ppm | 381 min |
| EMIM - Ethylsulfat / Proglyme | 50 ppm | PTZ | 20 ppm | 352 min |

(fortgesetzt)

| Additiv | | Inhibitor | | Inhibierungsperiode |
|---|---|---|---|---|
| LM, IL oder IL/LM | Konzentration | Verbindung | Konzentration | |
| | ppm | | ppm | min |
| TBA - Ethercarboxylat | 22 ppm | PTZ | 20 ppm | 378 min |
| TBA - Ethercarboxylat / Methylbutylketon | 50 ppm | PTZ | 20 ppm | 336 min |
| TBA - Ethercarboxylat / Morpholin | 33 ppm | PTZ | 20 ppm | 344 min |
| TBA - Ethercarboxylat / Agnique AMD 10 | 1000 ppm | PTZ | 20 ppm | 353 min |
| TBA - Ethercarboxylat / Ethylendiamin | 1000 ppm | PTZ | 20 ppm | 366 min |
| TBA - Ethercarboxylat / 3-(Dimethylamino)-1-propylamin | 1000 ppm | PTZ | 20 ppm | 440 min |
| TBA - Ethercarboxylat / N-Methylimidazol | 1000 ppm | PTZ | 20 ppm | 416 min |
| TBA - Ethercarboxylat / Proglyme | 50 ppm | PTZ | 20 ppm | 338 min |
| Pluriol A500 E = Methylpolyethylenglykol,<br>Pluriol E 400 = Polyethylenglykol;<br>Dowtherm A = eutektische Mischung von Diphenyl und Diphenyloxid;<br>Agnique AMD 3L = Milchsäuredimethylamid;<br>Agnique FOH 9 OC = Oleyl-/Cetylfettalkohol;<br>Agnique FOH 898 = Caprylalkohol;<br>Agnique AE 32 EH =2-Ethylhexyllactat. | | | | |

Beispiel 3

Die Löslichkeit von Phenothiazin in Gemischen von 20 Gew.-% ionischer Flüssigkeit und 80 Gew.-% Lösungsmittel wurde bestimmt, indem die Lösungen bei Zimmertemperatur nach und nach mit Phenothiazin versetzt wurden, bis ein deutlicher Phenothiazin-Niederschlag zu erkennen war. Auf gleiche Weise wurde die Löslichkeit von Phenothiazin im reinen Lösungsmittel bestimmt.

Folgende Lösungsmittel wurden untersucht: Dimethylsulfat (DMSO), Sulfolan, Entsalztes Wasser, Benzonitril, Essigsäure, Propionsäure, Dimethylsuccinat, Methylbenzoat, γ-Butyrolacton, Acetonitril, Cyclohexanon, Methylbutylketon, Morpholin, Ethylenglycol, Methylpolyethylenglycol Mw 500 (Pluriol A 500 E), Polyethylenglycol Mw 400 (Pluriol E 400), Milchsäure-dimethylamid (Agnique AMD 3 L), Dibutyladipat (Cetiol B), Di-(2-ethylhexyl)adipat (Plastomoll DOA), n-Octanol (Agnique FOH 898), C10-Fettsäuredimethylamid (Agnique AMD 10), Ethylendiamin, 3-(Dimethylamino)-1-propylamin, N-Methylimidazol, Dimethylethylenharnstoff, 2-Ethylhexyllactat (Agnique AE 3-2 EH), Benzylalkohol, Dimethylphthalat, ein eutektisches Gemisch von Biphenyl und Diphenyloxid (Dowtherm A), Anisacetal, Decahydronaphthalin, Oleyl/Cetyl-Fettalkohol (Agnique FOH 9 OC), Dipropylenglycol-dimethylether (Proglyme).

Die relativen Löslichkeiten von Phenothiazin in einem Gemisch von ionischer Flüssigkeit und Lösungsmittel, bezogen auf die Löslichkeit von Phenothiazin im reinen Lösungsmittel, sind in den Figuren 1 bis 4 dargestellt. Man sieht, dass mit protischen Lösungsmitteln wie Essigsäure, Propionsäure, Wasser, Ethylenglycol, Ethylendiamin nur ein geringer löslichkeitserhöhender Effekt bzw. ein löslichkeitsvermindernder Effekt zu beobachten ist.

Beispiel 4

Es wurde das Phasendiagramm (Dreiecksdiagramm) des Systems Phenothiazin/Proglyme/EMIM Acetat bei Zimmertemperatur bestimmt. Das Phasendiagramm ist in Figur 5 dargestellt. Im Dreiecksdiagramm entsprechen die Ecken den reinen Stoffen. Die Schenkel des Dreiecks entsprechen Zweistoff-Gemischen. Die Anteile von Phenothiazin/Proglyme/EMIM Acetat an einem beliebigen Punkt P sind über die Schnittpunkte der Schenkelparallelen durch P mit den anderen Schenkeln gegeben. Das Phasenverhalten (einphasig, zwei Phasen flüssig/flüssig, zwei Phasen

fest/flüssig, dreiphasig) an ausgewählten Punkten ist durch Symbole angegeben.

Man sieht, dass bereits geringe Zusätze der ionischen Flüssigkeit die Löslichkeit des Phenothiazins im Lösungsmittel erhöhen, wobei ein linearer Anstieg bis zur Löslichkeit in der reinen ionische Flüssigkeit beobachtet wird. Man sieht außerdem, dass eine Inkompatibilität zwischen der ionischen Flüssigkeit und dem Lösungsmittel (Bildung zweier flüssiger Phasen) durch den Zusatz von Phenothiazin ab einer bestimmten Konzentration aufgehoben wird.

Beispiel 5

Es wurde das Phasendiagramm (Dreiecksdiagramm) des Systems Phenothiazin/Proglyme/EMIM Ethylsulfat bei Zimmertemperatur bestimmt. Das Phasendiagramm ist in Figur 6 dargestellt. Im Dreiecksdiagramm entsprechen die Ecken den reinen Stoffen. Die Schenkel des Dreiecks entsprechen Zweistoff-Gemischen. Die Anteile von Phenothiazin/Proglyme/EMIM Ethylsulfat an einem beliebigen Punkt P sind über die Schnittpunkte der Schenkelparallelen durch P mit den anderen Schenkeln gegeben. Das Phasenverhalten (einphasig, zwei Phasen flüssig/flüssig, zwei Phasen fest/flüssig, dreiphasig) an ausgewählten Punkten ist durch Symbole angegeben.

Man sieht, dass bereits geringe Zusätze der ionischen Flüssigkeit die Löslichkeit des Phenothiazins im Lösungsmittel erhöhen, wobei ein linearer Anstieg bzw. ein leichter synergistischer Effekt bis zur Löslichkeit in der reinen ionische Flüssigkeit beobachtet wird. Man sieht außerdem, dass eine Inkompatibilität zwischen der ionischen Flüssigkeit und dem Lösungsmittel (Bildung zweier flüssiger Phasen) durch den Zusatz von Phenothiazin ab einer bestimmten Konzentration aufgehoben wird.

## Patentansprüche

1. Zusammensetzung zur Sofortbeendigung einer radikalischen Polymerisation, umfassend

   a) einen unter Phenothiazinen ausgewählten Inhibitor der radikalischen Polymerisation,
   b) ein aprotisches Lösungsmittel und
   c) eine ionische Flüssigkeit.

2. Zusammensetzung nach Anspruch 1, wobei die Zusammensetzung wenigstens 20 Gew.-% Inhibitor, bezogen auf das Gesamtgewicht der Komponenten a), b) und c) enthält.

3. Zusammensetzung nach einem der vorhergehenden Ansprüche, wobei das Gewichtsverhältnis von aprotischem Lösungsmittel zu ionischer Flüssigkeit im Bereich von 100 : 1 bis 1 : 10 liegt.

4. Zusammensetzung nach einem der vorhergehenden Ansprüche, wobei das aprotische Lösungsmittel eine relative statische Permittivität $\varepsilon_r$ als flüssige Reinsubstanz bei einer Temperatur von 293,15 K und einem Druck von $1{,}0133 \cdot 10^5$ Pa im Bereich von 3 bis 50, vorzugsweise 5 bis 38, aufweist.

5. Zusammensetzung nach einem der Ansprüche 1 bis 3, wobei das aprotische Lösungsmittel eine Lage im Hansen-Löslichkeitsraum aufweist, die **gekennzeichnet ist durch**

$$\sqrt{4(\delta_D - 17)^2 + (\delta_P - 11)^2 + (\delta_H - 6)^2} \leq 9.$$

6. Zusammensetzung nach einem der vorhergehenden Ansprüche, wobei der Inhibitor Phenothiazin ist.

7. Zusammensetzung nach einem der vorhergehenden Ansprüche, wobei das aprotische Lösungsmittel unter Kohlenwasserstoffen, Ethern, Estern, Amiden, Nitrilen, Acetalen oder Mischungen davon ausgewählt ist.

8. Zusammensetzung nach einem der vorhergehenden Ansprüche, wobei die ionische Flüssigkeit ein unter quartären Ammonium-, Oxonium-, Sulfonium- Phosphonium- Uronium-, Thiouronium- und Guanidinium-Kationen ausgewähltes organisches Kation umfasst.

9. Zusammensetzung nach Anspruch 8, wobei das organische Kation ausgewählt ist unter quartären Ammoniumionen der Formel (II)

$N^{+}R^{1}R^{2}R^{3}R^{4}$ (II),

worin $R^1$, $R^2$, $R^3$ und $R^4$ unabhängig voneinander für Alkylreste stehen, die mit heterofunktionellen Gruppen substituiert sein können und die untereinander aliphatische Ringsysteme bilden können,
Imidazoliumionen der Formel (III)

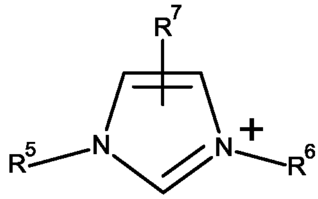

(III),

worin $R^5$ für Wasserstoff oder Alkyl steht, $R^6$ für Alkyl steht und $R^7$ für Wasserstoff oder Alkyl steht,
N-substituierten Pyridiniumderivaten,
N,N'-di substituierten Pyrazoliumderivaten und
Guadiniumderivaten.

**10.** Zusammensetzung nach einem der Ansprüche 1 bis 8, wobei die ionische Flüssigkeit ein unter Carboxylaten, Sulfonaten, Phosphonaten, Halogeniden, Bis(trifluorsulfon)imiden, Aluminiumtetrachlorid, Phosphorfluorid oder Dicyanimiden ausgewähltes Anion umfasst.

**11.** Zusammensetzung nach Anspruch 10, wobei das Anion unter Alkylcarboxylaten, Polyether-haltigen Carboxylaten oder Alkylsulfonaten ausgewählt ist.

**12.** Verwendung der Zusammensetzung gemäß einem der vorhergehenden Ansprüche zur Stabilisierung von radikalisch polymerisierbaren Monomeren gegen radikalische Polymerisation.

**13.** Verwendung nach Anspruch 12, wobei die radikalisch polymerisierbaren Monomere ausgewählt sind unter 1-Olefinen, Vinylhalogeniden, Vinylaromaten, Vinylethern, Vinylestern, Vinylamiden, Vinylcarbonsäuren, Vinylaromatcarbonsäuren, Acrylsäuren, Acrylsäureestern, Acrylamiden, Acrylnitrilen und Allylestern.

**14.** Verfahren zur Sofortbeendigung von radikalischen Polymerisationen, wobei man die Zusammensetzung nach einem der Ansprüche 1 bis 11 einem radikalisch polymerisierenden System zusetzt.

**15.** Verfahren nach Anspruch 14, wobei

(i) der Beginn einer unkontrollierten radikalischen Polymerisation in dem System registriert wird, wenn der Temperaturanstieg des Systems pro Zeit über einem vorgegebenen Wert liegt, und
(ii) die Zusammensetzung nach einem der Ansprüche 1 bis 11 in das radikalisch polymerisierende System eingebracht wird.

## Claims

**1.** A composition for the immediate stopping of a free-radical polymerization, comprising

a) a free-radical polymerization inhibitor selected from among phenothiazines,
b) an aprotic solvent and
c) an ionic liquid.

**2.** The composition according to claim 1, wherein the composition comprises at least 20% by weight of inhibitor, based on the total weight of the components a), b) and c).

**3.** The composition according to any of the preceding claims, wherein the weight ratio of aprotic solvent to ionic liquid is in the range from 100:1 to 1:10.

4. The composition according to any of the preceding claims, wherein the aprotic solvent has a relative static permittivity $\varepsilon_r$ as liquid pure substance at a temperature of 293.15 K and a pressure of $1.0133 \cdot 10^5$ Pa in the range from 3 to 50, preferably from 5 to 38.

5. The composition according to any of claims 1 to 3, wherein the aprotic solvent has a position in the Hansen solubility space which is such that

$$\sqrt{4(\delta_D - 17)^2 + (\delta_P - 11)^2 + (\delta_H - 6)^2} \leq 9.$$

6. The composition according to any of the preceding claims, wherein the inhibitor is phenothiazine.

7. The composition according to any of the preceding claims, wherein the aprotic solvent is selected from among hydrocarbons, ethers, esters, amides, nitriles, acetals and mixtures thereof.

8. The composition according to any of the preceding claims, wherein the ionic liquid comprises an organic cation selected from among quaternary ammonium, oxonium, sulfonium, phosphonium, uronium, thiouronium and guanidinium cations.

9. The composition according to claim 8, wherein the organic cation is selected from among quaternary ammonium ions of the formula (II)

$$N^+R^1R^2R^3R^4 \qquad (II),$$

where $R^1$, $R^2$, $R^3$ and $R^4$ are, independently of one another, alkyl radicals which may be substituted by heterofunctional groups and can form aliphatic ring systems with one another,
imidazolium ions of the formula (III)

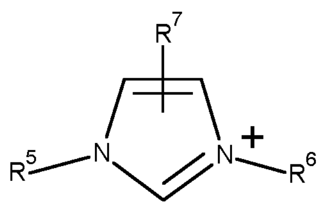

(III),

where $R^5$ is hydrogen or alkyl, $R^6$ is alkyl and $R^7$ is hydrogen or alkyl,
N-substituted pyridinium derivatives,
N,N'-disubstituted pyrazolium derivatives and guadinium derivatives.

10. The composition according to any of claims 1 to 8, wherein the ionic liquid comprises an anion selected from among carboxylates, sulfonates, phosphonates, halides, bis(trifluorosulfonyl)imides, aluminum tetrachloride, phosphorus fluoride and dicyanimides.

11. The composition according to claim 10, wherein the anion is selected from among alkylcarboxylates, polyether-comprising carboxylates and alkylsulfonates.

12. The use of the composition according to any of the preceding claims for the stabilization of free-radically polymerizable monomers against free-radical polymerization.

13. The use according to claim 12, wherein the free-radically polymerizable monomers are selected from among 1-olefins, vinyl halides, vinylaromatics, vinyl ethers, vinyl esters, vinylamides, vinylcarboxylic acids, vinylaromaticcarboxylic acids, acrylic acids, acrylic esters, acrylamides, acrylonitriles and allyl esters.

14. A method for the immediate stopping of free-radical polymerizations, wherein the composition according to any of claims 1 to 11 is added to a free-radically polymerizing system.

**15.** The method according to claim 14, wherein

(i) the commencement of an uncontrolled free-radical polymerization in the system is registered when the temperature increase of the system per unit time is above a prescribed value and
(ii) the composition according to any of claims 1 to 11 is introduced into the free-radically polymerizing system.

**Revendications**

**1.** Composition de cessation immédiate d'une polymérisation radicalaire, comprenant

a) un inhibiteur de la polymérisation radicalaire choisie parmi des phénothiazines,
b) un solvant aprotique et
c) un liquide ionique.

**2.** Composition selon la revendication 1, la composition contenant au moins 20 % massiques d'inhibiteur, en référence au poids total des composantes a), b) et c).

**3.** Composition selon l'une des revendications précédentes, le rapport massique entre le solvant aprotique et le liquide ionique étant compris dans la plage de 100:1 à 1:10.

**4.** Composition selon l'une des revendications précédentes, le solvant aprotique, en tant que substance liquide pure à une température de 293,15 K et une pression de $1{,}0133 \cdot 10^5$ Pa, possédant une permittivité statique relative $\varepsilon_r$ comprise dans la plage de 3 à 50, de préférence de 5 à 38.

**5.** Composition selon l'une des revendications 1 à 3, le solvant aprotique possédant une couche dans l'espace de solubilité de Hansen, laquelle est **caractérisée par**

$$\sqrt{4(\delta_D - 17)^2 + \left(\delta_p - 11\right)^2 + (\delta_H - 6)^2} \leq 9\,.$$

**6.** Composition selon l'une des revendications précédentes, l'inhibiteur étant de la phénothiazine.

**7.** Composition selon l'une des revendications précédentes, le solvant aprotique étant choisi parmi les hydrocarbures, les éthers, les esters, les amides, les nitriles, les acétals ou des mélanges de ceux-ci.

**8.** Composition selon l'une des revendications précédentes, le liquide ionique comprenant un cation organique choisi parmi les cations quaternaires d'ammonium, d'oxonium, de sulfonium, de phosphonium, d'uronium, de thiouronium et de guanidinium.

**9.** Composition selon la revendication 8, le cation organique étant choisi parmi les ions d'ammonium quaternaires de la formule (II)

$$N^+R^1R^2R^3R^4 \qquad (II),$$

$R^1$, $R^2$, $R^3$ et $R^4$ désignant des résidus d'alkyle indépendants l'un de l'autre qui peuvent être substitués avec des groupes hérérofonctionnels et qui peuvent former entre eux des systèmes cycliques aliphatiques,
les ions d'imidazole de la formule (III)

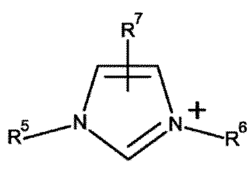

(III),

$R^5$ désignant l'hydrogène ou un alkyle, $R^6$ un alkyle et $R^7$ l'hydrogène ou un alkyle,
des dérivés de pyridinium substitués N,
des dérivés de pyrazolium substitués N,N'-di et
des dérivés de guadinium.

**10.** Composition selon l'une des revendications 1 à 8, le liquide ionique comprenant un anion choisi parmi les carboxylates, les sulfonates, les phosphonates, les halogénides, les bis(trifluorosulfone)imides, le tétrachlorure d'aluminium, le fluorure de phosphore ou les dicyanimides.

**11.** Composition selon la revendication 10, l'anion étant choisi parmi les alkylcarboxylates, les carboxylates contenant du polyéther ou les alkylsulfonates.

**12.** Utilisation de la composition selon l'une des revendications précédentes pour la stabilisation de monomères polymérisables de manière radicalaire contre la polymérisation radicalaire.

**13.** Utilisation selon la revendication 12, les monomères polymérisables de manière radicalaire étant choisis parmi les 1-oléfines, les halogénures de vinyle, les aromates de vinyle, les éthers de vinyle, les esters de vinyle, les amides de vinyle, les acides carboniques de vinyle, les acides carboniques d'aromate de vinyle, les acides acryliques, les esters d'acide acrylique, les acrylamides, les acrylonitriles et les esters allyliques.

**14.** Procédé de cessation immédiate de polymérisations radicalaires, selon lequel la composition selon l'une des revendications 1 à 11 est ajoutée à un système à polymérisation radicalaire.

**15.** Procédé selon la revendication 14,

(i) le début d'une polymérisation radicalaire non contrôlée dans le système est enregistré lorsque l'augmentation de température du système par unité de temps est supérieure à une valeur prédéfinie, et
(ii) la composition selon l'une des revendications 1 à 11 est introduite dans le système à polymérisation radicalaire.

FIG. 1

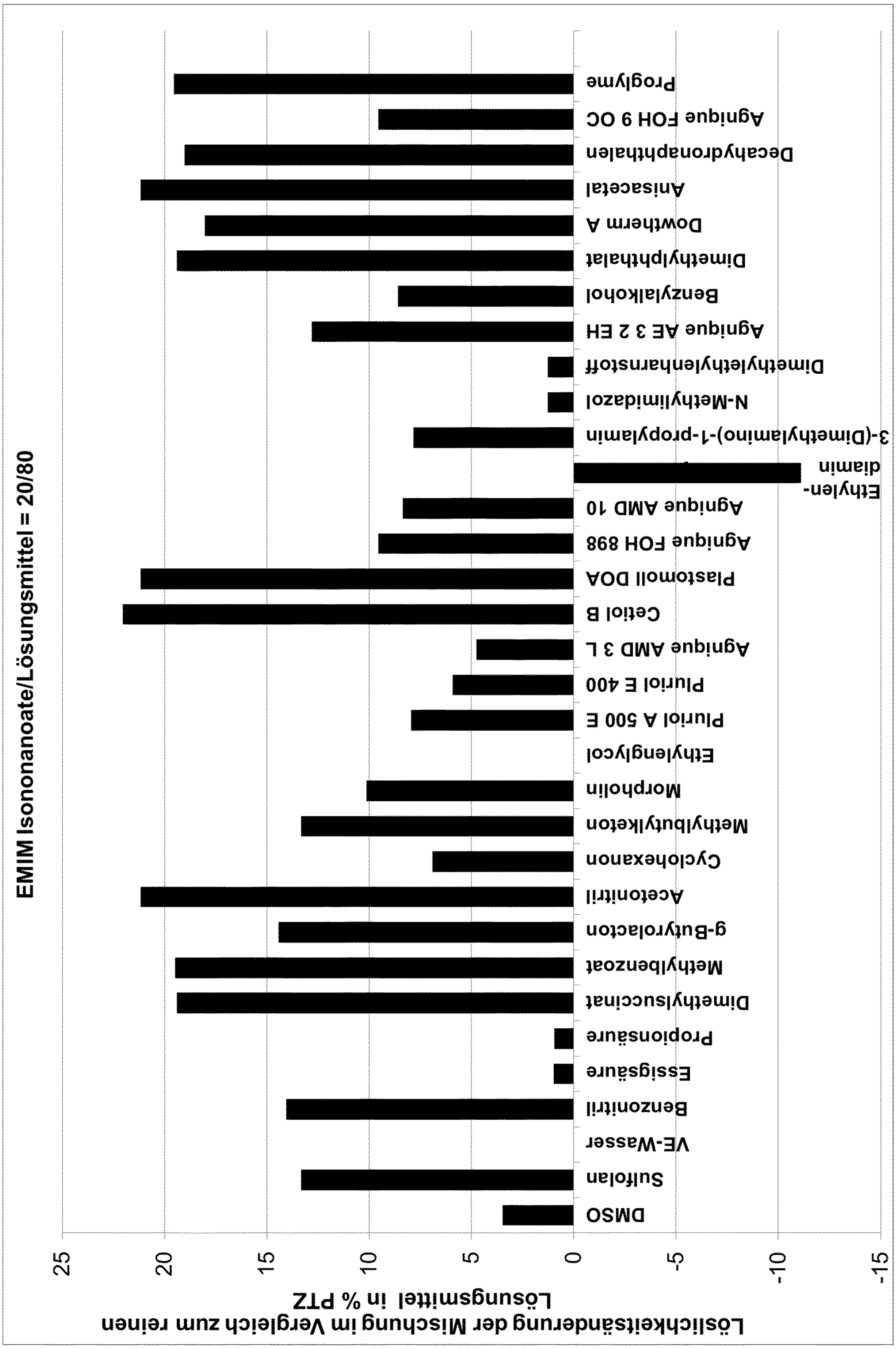
EMIM Isononanoate/Lösungsmittel = 20/80
Löslichkeitsänderung der Mischung im Vergleich zum reinen Lösungsmittel in % PTZ
25
20
15
10
5
0
-5
-10
-15
DMSO
Sulfolan
VE-Wasser
Benzonitril
Essigsäure
Propionsäure
Dimethylsuccinat
Methylbenzoat
g-Butyrolacton
Acetonitril
Cyclohexanon
Methylbutylketon
Morpholin
Ethylenglycol
Pluriol A 500 E
Pluriol E 400
Agnique AMD 3 L
Cetiol B
Plastomoll DOA
Agnique FOH 898
Agnique AMD 10
Ethylen-diamin
3-(Dimethylamino)-1-propylamin
N-Methylimidazol
Dimethylethylenharnstoff
Agnique AE 3 2 EH
Benzylalkohol
Dimethylphthalat
Dowtherm A
Anisacetal
Decahydronaphthalen
Agnique FOH 9 OC
Proglyme

**FIG. 2**

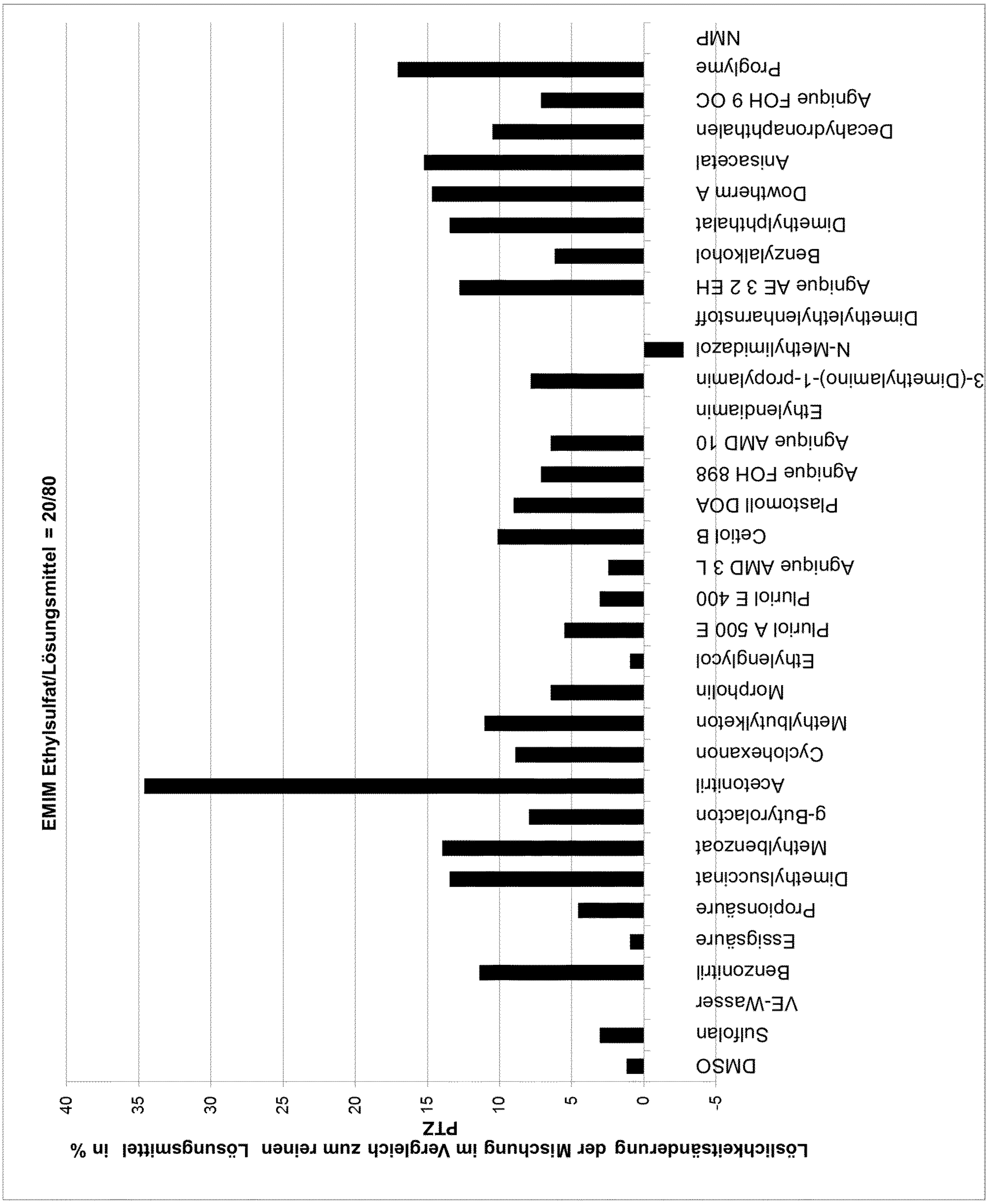
EMIM Ethylsulfat/Lösungsmittel = 20/80
Löslichkeitsänderung der Mischung im Vergleich zum reinen Lösungsmittel in %
PTZ
-5
0
5
10
15
20
25
30
35
40
DMSO
Sulfolan
VE-Wasser
Benzonitril
Essigsäure
Propionsäure
Dimethylsuccinat
Methylbenzoat
g-Butyrolacton
Acetonitril
Cyclohexanon
Methylbutylketon
Morpholin
Ethylenglycol
Pluriol A 500 E
Pluriol E 400
Agnique AMD 3 L
Cetiol B
Plastomoll DOA
Agnique FOH 898
Agnique AMD 10
Ethylendiamin
3-(Dimethylamino)-1-propylamin
N-Methylimidazol
Dimethylethylenharnstoff
Agnique AE 3 2 EH
Benzylalkohol
Dimethylphthalat
Dowtherm A
Anisacetal
Decahydronaphthalen
Agnique FOH 9 OC
Proglyme
NMP

FIG. 3

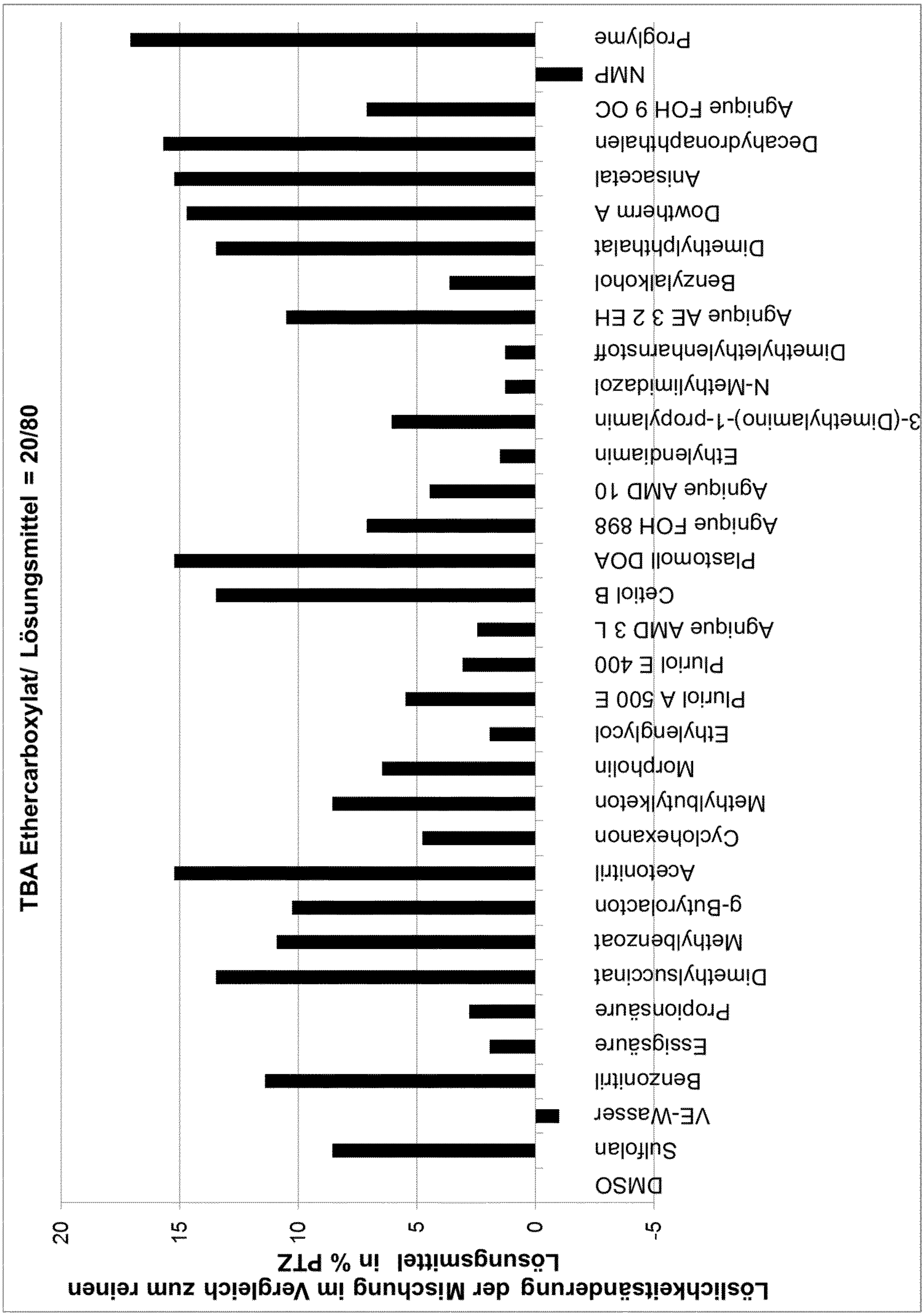
TBA Ethercarboxylat/ Lösungsmittel = 20/80
Löslichkeitsänderung der Mischung im Vergleich zum reinen Lösungsmittel in % PTZ
20
15
10
5
0
-5
DMSO
Sulfolan
VE-Wasser
Benzonitril
Essigsäure
Propionsäure
Dimethylsuccinat
Methylbenzoat
g-Butyrolacton
Acetonitril
Cyclohexanon
Methylbutylketon
Morpholin
Ethylenglycol
Pluriol A 500 E
Pluriol E 400
Agnique AMD 3 L
Cetiol B
Plastomoll DOA
Agnique FOH 898
Agnique AMD 10
Ethylendiamin
3-(Dimethylamino)-1-propylamin
N-Methylimidazol
Dimethylethylenharnstoff
Agnique AE 3 2 EH
Benzylalkohol
Dimethylphthalat
Dowtherm A
Anisacetal
Decahydronaphthalen
Agnique FOH 9 OC
NMP
Proglyme

FIG. 4

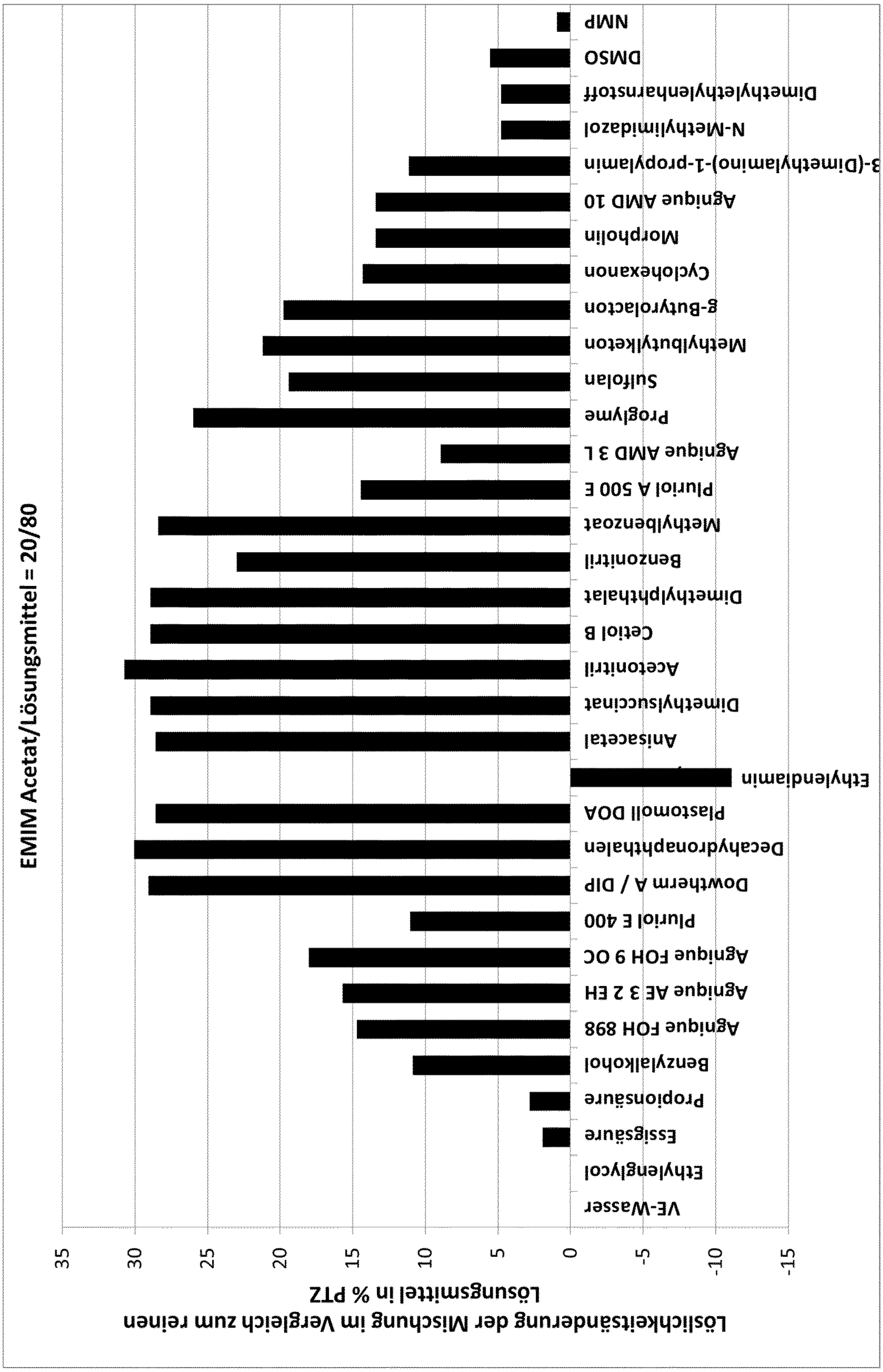
EMIM Acetat/Lösungsmittel = 20/80
Löslichkeitsänderung der Mischung im Vergleich zum reinen Lösungsmittel in % PTZ
35
30
25
20
15
10
5
0
-5
-10
-15
VE-Wasser
Ethylenglycol
Essigsäure
Propionsäure
Benzylalkohol
Agnique FOH 898
Agnique AE 3 2 EH
Agnique FOH 9 OC
Pluriol E 400
Dowtherm A / DIP
Decahydronaphthalen
Plastomoll DOA
Ethylendiamin
Anisacetal
Dimethylsuccinat
Acetonitril
Cetiol B
Dimethylphthalat
Benzonitril
Methylbenzoat
Pluriol A 500 E
Agnique AMD 3 L
Proglyme
Sulfolan
Methylbutylketon
g-Butyrolacton
Cyclohexanon
Morpholin
Agnique AMD 10
3-(Dimethylamino)-1-propylamin
N-Methylimidazol
Dimethylethylenharnstoff
DMSO
NMP

FIG. 5

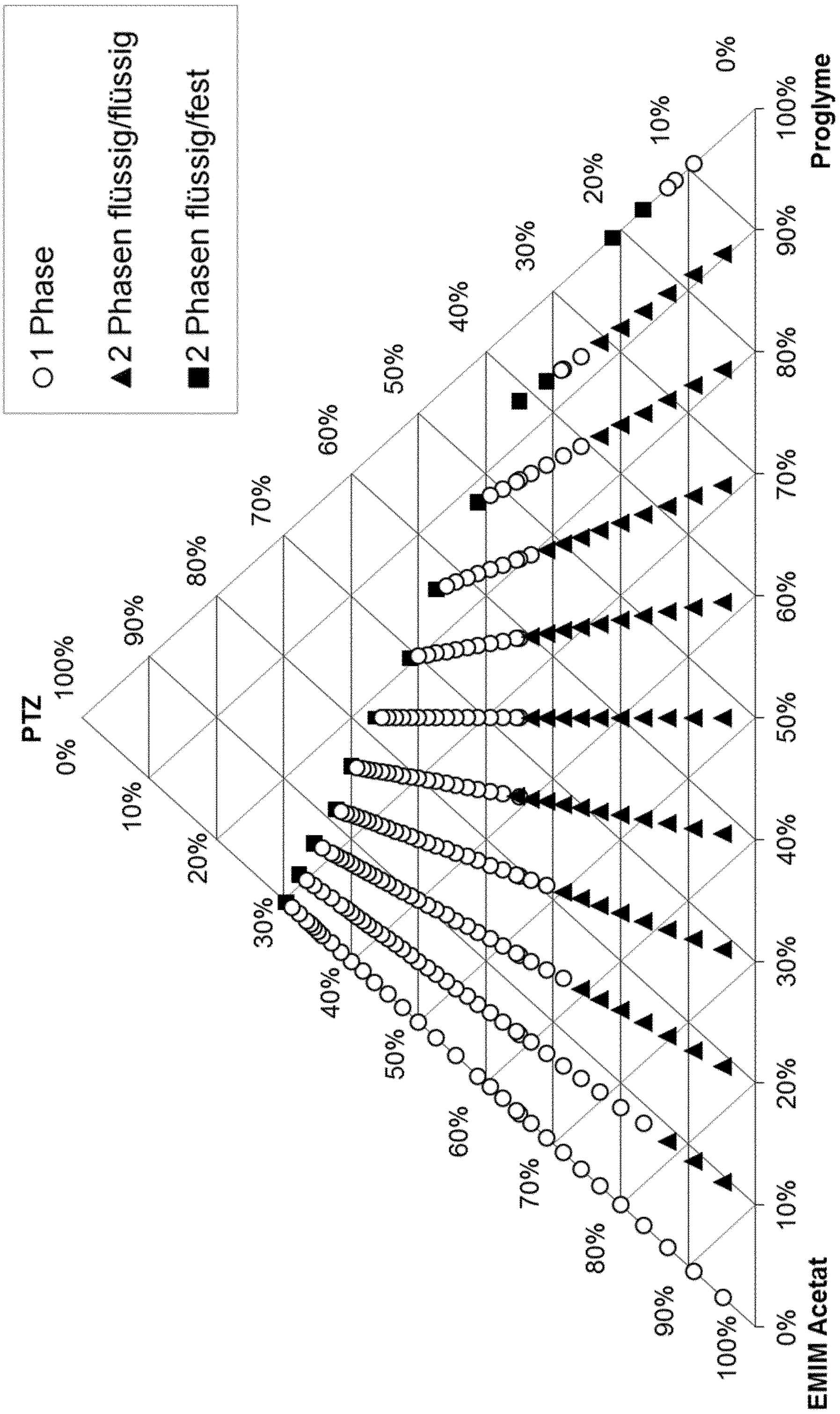

1 Phase
2 Phasen flüssig/flüssig
2 Phasen flüssig/fest
PTZ
Proglyme
EMIM Acetat
0%
10%
20%
30%
40%
50%
60%
70%
80%
90%
100%

**FIG. 6**

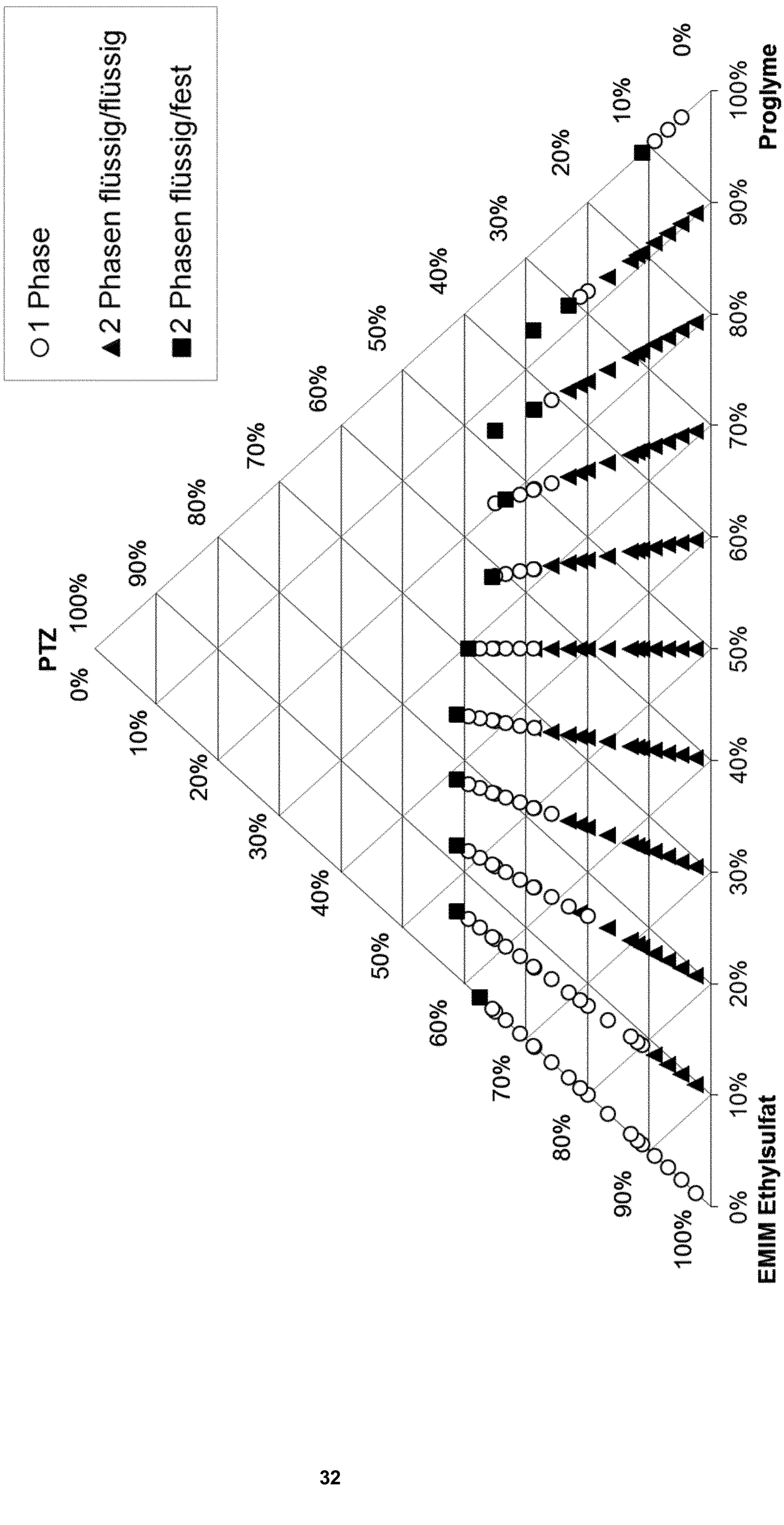
1 Phase
2 Phasen flüssig/flüssig
2 Phasen flüssig/fest
PTZ
Proglyme
EMIM Ethylsulfat
0%
10%
20%
30%
40%
50%
60%
70%
80%
90%
100%

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10036959 A1
- EP 2017293 A1
- WO 9921893 A
- DE 102005055815 A
- DE 102005035103 A1
- DE 10325050 A1
- WO 2008135482 A
- EP 135280 A
- DE 19651307 A
- US 5322912 A
- US 5412047 A
- US 4581429 A
- DE 1618141 A
- CN 1052847 A
- US 4670131 A
- US 5322960 A
- DE 19602539 A
- EP 765856 A
- JP 5320217 A
- US 4665185 A
- DE 19510184 A
- DE 102007055086 A


### In der Beschreibung aufgeführte Nicht-Patentliteratur


- Radikalische Polymerisation. *Römpp Online,* 2007
- Inhibierung. *Römpp Online,* 2007
- HANDBOOK of CHEMISTRY and PHYSICS. CRC PRESS, 2010
- **C. M. HANSEN.** The three dimensional solubility parameters. *J. Paint Technol.,* 1967, vol. 39, 105
- **C. M. HANSEN.** Hansen Solubility Parameters: A User's Handbook. 2007
- **C. M. HANSEN.** HSPIP 3.1.14
- **WASSERSCHEID ; WELTON.** Ionic liquids in synthesis. Wiley-VCH, 2007
- **ROMANELLI, M. ; OTTAVIANI, M.F. ; MARTINI, G. ; KEVAN, L., JPCH J.** *Phys. Chem., EN,* 1989, vol. 93 (1), 317-322
- Vinylmonomere. *Römpp Online,* 2007